(19) Europäisches Patentamt
European Patent Office
Office européen des brevets

(11) EP 1 295 587 A1

(12) # EUROPEAN PATENT APPLICATION
published in accordance with Art. 158(3) EPC

(43) Date of publication:
**26.03.2003 Bulletin 2003/13**

(21) Application number: **01917726.0**

(22) Date of filing: **30.03.2001**

(51) Int Cl.[7]: **A61K 7/00**, A61K 7/48, A61P 17/16

(86) International application number:
**PCT/JP01/02787**

(87) International publication number:
**WO 01/072265 (04.10.2001 Gazette 2001/40)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.03.2000 JP 2000097428**
**29.08.2000 JP 2000258995**
**28.12.2000 JP 2000403353**

(71) Applicant: **The Nisshin OilliO, Ltd.**
**Tokyo 104-8285 (JP)**

(72) Inventors:
- **KUNO, Noriyasu;**
**c/o THE NISSHIN OIL MILLS, LTD.**
**Yokosuka-shi, Kanagawa 239-0832 (JP)**
- **SHINOHARA, Gou;**
**c/o THE NISSHIN OIL MILLS, LTD.**
**Yokosuka-shi, Kanagawa 239-0832 (JP)**

(74) Representative: **Bawden, Peter Charles et al**
**Bawden & Associates,**
**4 The Gatehouse**
**2 High Street**
**Harpenden, Hertfordshire AL5 2TH (GB)**

(54) **EXTERNAL PREPARATION FOR THE SKIN AND BEAUTIFYING AGENTS**

(57) The present invention relates to an external agent for the skin comprising at least one member selected from the group consisting of maslinic acid, erythrodiol, uvaol, betulinic acid, betulin, physiologically acceptable salts thereof and derivatives thereof.

EP 1 295 587 A1

## Description

### Technical Field

The present invention relates to an external agent for the skin and more particularly to an external agent for the skin having a whitening effect as well as a whitening agent.

### Background of the Invention

The activity of melanocytes is in general stimulated by, for instance, the exposure of the skin to UV light rays included in the sun light, the paraeccrisis of hormones and genetic factors and the melanin thus synthesized in the melanocytes abnormally precipitates within the skin to thus cause the dark skin, melasma and ephelis of the skin. To relieve or lighten and/or prevent such abnormal precipitation of melanin, there have been developed a variety of whitening agents such as L-ascorbic acid or derivatives thereof, hydroquinone derivatives, glutathione and colloidal sulfur. However, various problems arise such that some of them have only a weak melanogenesis-inhibitory function, are quite unstable and susceptible to oxidation, give out nasty smells peculiar thereto and form precipitations. Moreover, they are insufficient in the whitening effect expected when they are individually incorporated into cosmetic products. Accordingly, there has still been desired for the development of whitening agents, which are highly effective, have a safe and stable whitening effect and also have an effect of preventing the occurrence of any melasma and ephelis.

On the other hand, maslinic acid is a kind of oleanane-type triterpenes and a compound having a structure represented by the structural formula 1. It is known that this compound shows an anti-inflammatory effect and an anti-histaminic effect. It is also known that the compound is present in nature and natural sources thereof are, for instance, olive, hop, mint, pomegranate, clove, sage and jujube.

In addition, erythrodiol is a kind of oleanane-type triterpenes and a compound having a structure represented by the structural formula 2. Up to now, there has been known that the compound has, for instance, an anti-inflammatory effect (see, Planta. Med., 1995, Vol. 61, No. 2, pp. 182-185). This is present in nature and examples of natural sources thereof include olive, sunflower, common marigold, gum Senegal and red sanders.

Moreover, uvaol is a kind of ursane-type triterpenes and has a structure represented by the structural formula 3 and the compound has been known to have, for instance, an anti-inflammatory effect (Planta. Med., 1995, Vol. 61, No. 2, pp. 182-185) and a glycerophosphoric acid dehydrogenase-inhibitory function (Japanese Un-Examined Patent Publication (hereunder referred to as "J.P. KOKAI") No. Hei 9-67249). It is known that the compound is present in nature and exists in, for instance, olive, bearberry, sage, gum Senegal and cajeput tree.

Betulinic acid is a kind of lupane-type triterpenes and have a structure represented by the structural formula 4 and it has been known that the compound shows a variety of functions such as a cancer-inhibitory function, an anti-inflammatory function, a wound-healing promoting function (Japanese Examined Patent Publication (hereunder referred to as "J.P. KOKOKU") No. Hei 4-26623), an alcohol absorption- inhibitory function (J.P. KOKAI No. Hei 7-53385) and a hair growth-promoting function (J.P. KOKAI No. Hei 9-157139). It is known that this compound is present in nature and examples of natural resources thereof are gentiana japonica Shult, clove, the rind of grape and olive, in which it is present in the free state, and Panacls Japonici Rhizoma, carrot and sugar beet wherein it is present as saponin.

Further, betulin is a kind of lupane-type triterpenes and has a structure represented by the structural formula 5 and it has been known that the compound has a variety of functions such as a biological protein modification-inhibitory function (J.P. KOKAI No. Hei 9-67253), a glycerophosphoric acid dehydrogenase-inhibitory function (J.P. KOKAI No. Hei 9-67249), a lipase-inhibitory function (J.P. KOKAI No. Hei 10-265328) and a liver disease-prophylactic function (J. P. KOKAI No. Hei 11-209275). It is known that the compound exists in nature, for instance, in the bark of white birch.

However, there has never been known that maslinic acid, erythrodiol, uvaol, betulinic acid, betulin and/or physiologically acceptable salts thereof or derivatives thereof show considerably excellent whitening effects such as the effects of preventing melasma and ephelis.

### Disclosure of the Invention

Accordingly, it is an object of the present invention to provide, for instance, an external agent for the skin having an excellent whitening effect and a whitening agent. It is another object of the present invention to provide, for instance, an external agent for the skin having not only an excellent whitening effect, but also high safety to the skin and a whitening agent.

The inventors of this invention have conducted screening of olive plants, an extract thereof and a purified product thereof using the whitening effect as an indication in order to achieve the foregoing objects. As a result, the inventors have found that maslinic acid and/or physiologically acceptable salts thereof have an excellent whitening

effect and have thus completed the present invention. Moreover, the inventors have likewise conducted inspection of substances having structures similar to those observed for these compounds for the presence of such a whitening effect, have found that specific substances belonging to the five-membered ring-containing triterpenes possess a desired whitening effect and have thus completed the present invention.

More specifically, the present invention relates to an external agent for the skin comprising at least one member selected from the group consisting of five-membered ring-containing triterpenes such as maslinic acid, erythrodiol, uvaol, betulinic acid, betulin and physiologically acceptable salts thereof or derivatives thereof and preferably an external agent for the skin comprising, as a whitening component, at least one member selected from the group consisting of five-membered ring-containing triterpenes such as maslinic acid, erythrodiol, uvaol, betulinic acid, betulin and physiologically acceptable salts thereof or derivatives thereof as well as an external agent for the skin comprising at least one member selected from the group consisting of five-membered ring-containing triterpenes such as maslinic acid, erythrodiol, uvaol, betulinic acid, betulin and physiologically acceptable salts thereof or derivatives thereof and at least one pharmaceutically active component selected from the group consisting of whitening agents, antioxidants, anti-inflammatory agents, cell activators, ultraviolet light absorbers, blood circulation promoting agents and humectants.

The present invention also relates to an external agent for the skin comprising maslinic acid and/or a physiologically acceptable salt thereof and preferably an external agent for the skin comprising them as whitening components. The present invention likewise relates to an external agent for the skin preferably comprising maslinic acid and/or a physiologically acceptable salt thereof as well as at least one pharmaceutically active component selected from the group consisting of whitening agents, antioxidants, anti-inflammatory agents, cell activators, ultraviolet light absorbers, blood circulation promoting agents and humectants.

Moreover, the present invention relates to a whitening agent comprising, as an effective component, at least one member selected from the group consisting of maslinic acid, erythrodiol, uvaol, betulinic acid, betulin and physiologically acceptable salts thereof or derivatives thereof as well as an external agent for the skin comprising the whitening agent.

In this respect, these maslinic acid, erythrodiol, uvaol, betulinic acid and betulin used in the present invention are substances represented by the following structural formulas 1 to 5, respectively and the term "physiologically acceptable salt(s) thereof" used herein means salts derived from the groups: -COOH of specific penta-cyclic triterpenes and includes those included in isolates from naturally occurring products by nature. The kinds of salts thereof are not particularly restricted inasmuch as they may usually be used in cosmetic or pharmaceutical compositions. Further, the term "derivative(s)" herein used means one capable of being biochemically or artificially formed and they are not restricted to particular ones inasmuch as they can be formed biochemically or artificially.

The maslinic acid and/or physiologically acceptable salts thereof, which may be incorporated into the external agent for the skin and the whitening agent according to the present invention are preferably those derived from olive, hop, mint, pomegranate, clove, sage and jujube and in particular, a product obtained by the extraction of olive plant and/or a product prepared during the olive oil-production processes with water and/or an organic solvent and subsequent concentration and/or fractionation- purification treatments.

The present invention preferably relates to a method for using, as an external agent for the skin, at least one member selected from the group consisting of maslinic acid, erythrodiol, uvaol, betulinic acid, betulin and physiologically acceptable salts thereof or derivatives thereof and a method for using, as a whitening agent, at least one member selected from the group consisting of maslinic acid, erythrodiol, uvaol, betulinic acid, betulin and physiologically acceptable salts thereof or derivatives thereof.

The present invention also relates to a method for using a compound selected from the group consisting of maslinic acid, erythrodiol, uvaol, betulinic acid, betulin and physiologically acceptable salts thereof or derivatives thereof, as an external agent for the skin or a whitening agent.

The present invention likewise relates to a melanogenesis-inhibitory agent comprising a compound selected from the group consisting of maslinic acid, erythrodiol, uvaol, betulinic acid, betulin and physiologically acceptable salts thereof or derivatives thereof.

Further, the present invention relates to a method for preventing the dark skin, melasma, ephelis and darkening or dullness of the skin through the use of a compound selected from the group consisting of maslinic acid, erythrodiol, uvaol, betulinic acid, betulin and physiologically acceptable salts thereof or derivatives thereof.

The present invention also relates to the use of a compound selected from the group consisting of maslinic acid, erythrodiol, uvaol, betulinic acid, betulin and physiologically acceptable salts thereof or derivatives thereof, as an external agent for the skin, a whitening agent or a melanogenesis-inhibitory agent.

The present invention likewise relates to a raw material for a whitening agent comprising a compound selected from the group consisting of maslinic acid, erythrodiol, uvaol, betulinic acid, betulin and physiologically acceptable salts thereof or derivatives thereof as well as the use of these compounds as raw materials for whitening agents.

Moreover, the present invention also relates to a method for preparing an external agent for the skin comprising maslinic acid, a physiologically acceptable salt thereof and/or a derivative thereof, which comprises the steps of derivatizing, if desired, maslinic acid, a physiologically acceptable salt thereof and/or a derivative thereof prepared by the extraction of olive plant and/or a product prepared during the olive oil-production processes with water and/or an organic solvent and subsequent concentration and/or fractionation-purification treatments; and

admixing the resulting product with at least one oil component or medicinal oil and at least one surfactant.

The present invention also relates to a method for preparing a toilet water, which comprises maslinic acid, a physiologically acceptable salt thereof and/or a derivative thereof, comprising the step of admixing at least one alcohol with maslinic acid, a physiologically acceptable salt thereof and/or a derivative thereof prepared by the extraction of olive plant and/or a product prepared during the olive oil-production processes with water and/or an organic solvent and subsequent concentration and/or fractionation-purification treatments.

<structural formula 1>

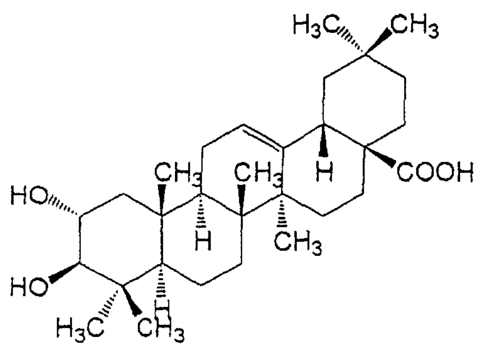

<structural formula 2>

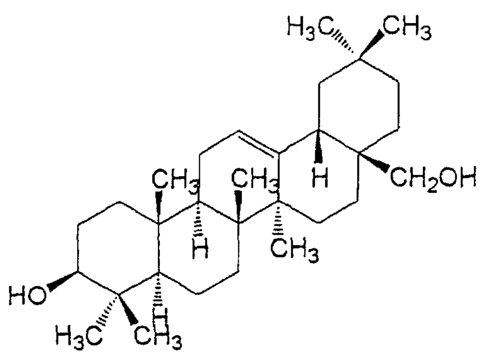

< structural formula 3 >

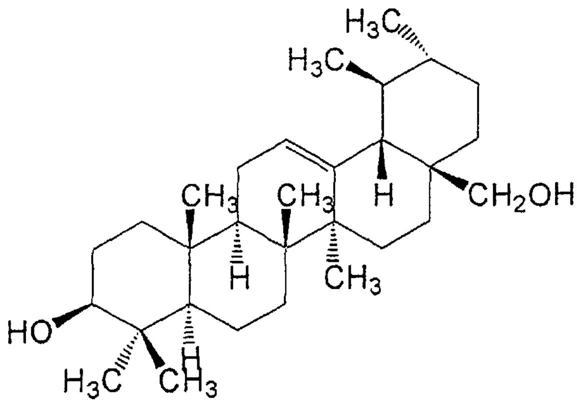

< structural formula 4 >

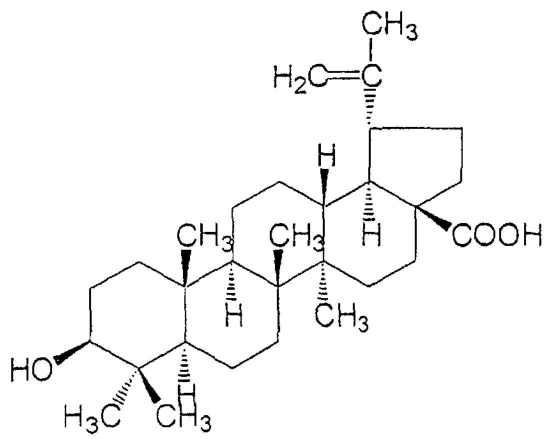

< structural formula 5 >

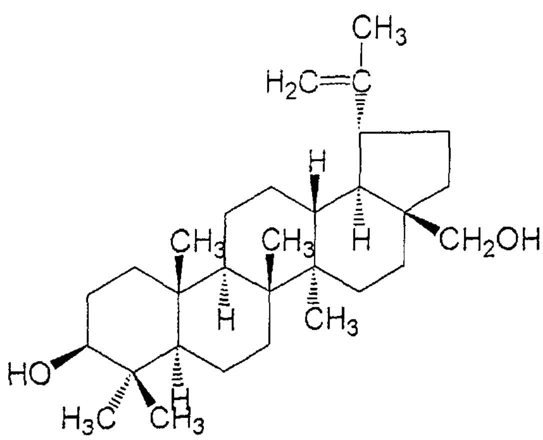

Best Mode for Carrying Out the Invention

The present invention will hereunder be described in more detail.

The present invention relates to an external agent for the skin comprising at least one member selected from the group consisting of penta-cyclic triterpenes such as maslinic acid, erythrodiol, uvaol, betulinic acid, betulin and physiologically acceptable salts thereof or derivatives thereof and preferably relates to an external agent for the skin comprising at least one member selected from the group consisting of maslinic acid, erythrodiol, uvaol, betulinic acid, betulin and physiologically acceptable salts thereof or derivatives thereof, as a whitening component.

In the present invention, the penta-cyclic triterpenes, or maslinic acid, erythrodiol, uvaol, betulinic acid, betulin and physiologically acceptable salts thereof or derivatives thereof are not restricted to particular ones and may be naturally occurring ones or those prepared through chemical synthesis, or those commercially available in the form of reagents. If they are ones derived from naturally occurring plants, they are preferred from the viewpoint of their excellent safety and freedom from anxiety. For instance, maslinic acid and/or physiologically acceptable salts thereof can be prepared from olive plant and/or products obtained during the processes for preparing olive oil and therefore, preferably used herein include external agents for the skin each comprising maslinic acid and/or a physiologically acceptable salt thereof or those each comprising, as a whitening agent, maslinic acid and/or a physiologically acceptable salt thereof. In this connection, the term "olive" used herein means olive plant and/or products obtained during the processes for preparing olive oil.

In this regard, the content of maslinic acid, erythrodiol, uvaol, betulinic acid, betulin and physiologically acceptable salts thereof or derivatives thereof, as penta-cyclic triterpenes in the external agent for the skin or the whitening agent is not restricted to any specific range, but preferably ranges from 0.00001 to 10% by mass. The term "physiologically acceptable salts" used herein means those derived from the group: -COOH present in specific penta-cyclic triterpenes and the kinds thereof are not restricted to specific ones inasmuch as they are commonly used in cosmetics or pharmaceutical compositions. In addition, the term "derivatives" used herein includes those capable of being biochemically or artificially formed and therefore, the derivatives are not restricted to any specific one in the present invention inasmuch as they can biochemically or artificially be prepared.

These penta-cyclic triterpenes, or maslinic acid, erythrodiol, uvaol, betulinic acid, betulin and physiologically acceptable salts thereof or derivatives thereof possess quite excellent whitening effects. For instance, according to one of cytologic evaluation methods using B16 melanoma cells as a means for the evaluation of the whitening effect, these substances are proved to possess a whitening effect about 5 to 200 times greater than that observed for vitamin C-magnesium phosphate, which is known as a substance having an excellent whitening effect. More specifically, the melanogenesis- inhibitory functions (whitening effects) of these substances are about 100 to 200 times for maslinic acid, about 100 to 200 times for salts of maslinic acid, about 10 to 100 times for erythrodiol, about 30 to 120 times for uvaol, about 30 to 120 times for betulinic acid, about 10 to 100 times for betulin, about 100 to 200 times for ethyl ester of maslinic acid, about 50 to 150 times for acetylated maslinic acid, about 30 to 120 times for triethyl silylated maslinic acid, about 50 to 150 times for ethyl ester of stearoyl-modified maslinic acid, about 10 to 100 times for acetylated erythrodiol, about 10 to 100 times for acetylated uvaol, about 10 to 100 times for ethyl ester of betulinic acid and about 5 to 50 times for acetylated betulin, as compared with that observed for the known substance. In other words, the use of penta-cyclic triterpenes and physiologically acceptable salts thereof or derivatives thereof would permit the impartment of their significantly strong melanogenesis-inhibitory functions to the external agent for the skin and whitening agent according to the present invention.

In the present invention, these agents may comprise at least one member selected from the group consisting of maslinic acid, erythrodiol, uvaol, betulinic acid, betulin and physiologically acceptable salts thereof or derivatives thereof and at least one pharmaceutically active component selected from the group consisting of whitening agents, antioxidants, anti-inflammatory agents, cell activators, ultraviolet light absorbers, blood circulation-promoting agents and humectants and the agents may arbitrarily be designed depending on various methods for using the same.

Moreover, the present invention relates to a whitening agent comprising, as an effective component, at least one member selected from the group consisting of maslinic acid, erythrodiol, uvaol, betulinic acid, betulin and physiologically acceptable salts thereof or derivatives thereof, which are penta-cyclic triterpenes. Moreover, the present invention relates, in particular, to a whitening agent comprising, as an effective component, maslinic acid and/or a physiologically acceptable salt thereof, as has been described above. These whitening agents can directly be applied to the skin to enjoy the whitening effect thereof on the skin and may likewise be used as raw materials for preparing the foregoing external agents for the skin.

As has been discussed above, maslinic acid, erythrodiol, uvaol, betulinic acid, betulin and physiologically acceptable salts thereof or derivatives thereof incorporated into the external agent for the skin and the whitening agent of the present invention may be prepared from naturally occurring plants and, in particular, maslinic acid and/or physiologically acceptable salts thereof can be prepared from olive, hop, mint, pomegranate, clove, sage, and jujube. In particular, if they are prepared from olive, they are favorably obtained by first extracting olive plant and/or products

obtained during the olive oil-production processes as raw materials with water and/or organic solvents and subsequently subjecting the resulting extracts to concentration and/or fractionation-purification treatments.

In this respect, the olive plant is preferably at least one member selected from the group consisting of fruits, seeds, rinds, leaves, stems, sprouts, or dried products, ground products and defatted products thereof and the products obtained during the olive oil-production processes are preferably at least one member selected from the group consisting of pressed residues, extraction residues, pressed oil, extracted oil, gum-free oil sludge, deacidified oil sludge, dark oil, waste discoloring agents, deodorized scum, pressed juice, waste water and waste filter mediums.

The present invention relates to an external agent for the skin and a whitening agent each comprising at least one member selected from the group consisting of maslinic acid, erythrodiol, uvaol, betulinic acid, betulin and physiologically acceptable salts thereof or derivatives thereof, as penta-cyclic triterpenes wherein the penta-cyclic triterpene is a kind of triterpenes or a penta-cyclic compound consisting of 6 isoprene units. In this connection, the carbon atom number of such a triterpene is fundamentally equal to 30, but may variously be changed during the biosynthetic processes through rearrangement, oxidation, elimination or alkylation.

These substances can be obtained from natural plants or artificially prepared. Alternatively, it is also convenient to use commercially available ones.

The penta-cyclic triterpenes are in general divided into subgroups on the basis of the skeletons thereof. For instance, subgroups thereof include oleanane-type triterpenes, ursane-type triterpenes, lupane-type triterpenes, hopane-type triterpenes, serratane-type triterpenes, friedelane-type triterpenes, taraxerane-type triterpenes, taraxastane-type triterpenes, multi-furolane-type triterpenes and germanicane-type triterpenes.

The inventors of this invention have found that specific penta-cyclic triterpenes or maslinic acid and erythrodiol as oleanane-type triterpenes, uvaol as an ursane-type triterpene, and betulinic acid and betulin as lupane-type triterpenes possess whitening effects such as an excellent melanogenesis-inhibitory function, among others and have thus completed the present invention. At the same time, the inventors have also found that other penta-cyclic triterpenes, whose skeletons are similar to those of the foregoing ones, such as oleanolic acid and β-amyrin as oleanane-type triterpenes, α-amyrin as an ursane-type triterpene, lupeol as a lupane-type triterpene never show any whitening effect (melanogenesis-inhibitory function) at all. In other words, it would be concluded that only specific substances among these penta-cyclic triterpenes show the desired effect of the present invention or the whitening effect and that such a whitening effect cannot be expected by the use of substances whose structures are simply resemblant to those observed for the substances discovered in the present invention. For instance, oleanolic acid and maslinic acid have structures quite similar to one another, but their whitening effects are considerably different from one another. In the present invention, the inventors have discovered or found out randomly existing substances showing whitening effects.

The present invention relates to an external agent for the skin and a whitening agent each comprising, as an effective component, at least one member selected from the group consisting of maslinic acid, erythrodiol, uvaol, betulinic acid, betulin and physiologically acceptable salts thereof or derivatives thereof and preferably relates to an external agent for the skin and a whitening agent each having a whitening effect such as a melanogenesis-inhibitory function.

In this respect, the term "physiologically acceptable salt(s)" herein used means salts derived from the carboxyl groups of specific penta-cyclic triterpenes (partial structure: -COOX; X represents an arbitrarily selected cationic substance), among others and these salts are not restricted to specific ones in the present invention inasmuch as they are commonly used in foods and beverages and pharmaceutical compositions and specific examples thereof include alkali metal salts such as sodium, potassium and lithium salts; alkaline earth metal salts such as calcium, magnesium, barium and zinc salts; ammonia; alkylamine salts such as methylamine, dimethylamine, trimethylamine, ethylamine, diethylamine, triethylamine, propylamine, butylamine, tetrabutylamine, pentylamine and hexylamine salts; alkanolamine salts such as ethanolamine, diethanolamine, triethanolamine, propanolamine, dipropanolamine, isopropanolamine and diisopropanolamine salts; other organic amine salts such as piperazine and piperidine salts; and basic amino acid salts such as salts with lysine, alginine, histidine and tryptophane. Among them, preferred are alkali metal salts, alkylamine salts, alkanolamine salts and basic amino acid salts. In general, these salts have water-solubility higher than those observed for the original specific penta-cyclic triterpenes from which they are derived and therefore, these salts are preferred, in particular, when they are used in an aqueous system.

In addition, the term "derivative(s)" used herein means ones capable of being biochemically or artificially prepared and the present invention includes all possible such derivatives, but specific examples thereof are derivatives carrying alcohol ester groups, those having fatty acid ester groups, those carrying alkoxy groups, those having alkoxymethyl groups or glycosides. Among these derivatives, those carrying alcohol ester groups, those having fatty acid ester groups, those carrying alkoxy groups and those having alkoxymethyl groups are highly oil-soluble as compared with the original specific penta-cyclic triterpenes from which they are derived and therefore, they are preferably used in the present invention, in particular, when they are applied to oil-containing systems, while the glycosides have water-solubility higher than those observed for the original specific penta-cyclic triterpenes from which they are derived and therefore, it is preferred in the present invention to use them in aqueous systems.

Some of these derivatives are also present in nature and can be obtained through the isolation thereof or they may likewise be prepared artificially. Moreover, the derivatives of the present invention can further be derivatized and salts thereof may likewise be used herein.

As has been discussed above, maslinic acid, erythrodiol, uvaol, betulinic acid and betulin can be converted into physiologically acceptable salts and derivatives thereof to thus improve the water-solubility or oil-solubility and accordingly, the present invention permits the arbitrary design of any product, which are improved in the handling characteristics, quality and whitening effects.

The alcohol ester group used herein means a functional group formed through the usual dehydration reaction between a carboxyl group and an alcoholic OH group (partial structure: -COOR in which R may be any hydrocarbon type functional group). In other words, the phrase "derivatives carrying alcohol ester group of penta-cyclic triterpenes" used herein means those capable of being formed from the carboxyl groups thereof with alcohols. In this respect, the alcohol is not restricted to any particular one, but specific examples thereof are methanol, ethanol, n-propanol, isopropanol, allyl alcohol, n-butanol, sec-butanol, tert-butanol, ethylene glycol, trimethylsilyl alcohol, triethylsilyl alcohol, phenol, benzyl alcohol and saccharides. Among them, preferred are derivatives derived from ethanol, triethylsilyl alcohol, methanol, n-propanol, isopropanol and trimethylsilyl alcohol.

The term "fatty acid ester group" used herein means a functional group formed as a result of the usual dehydration reaction of a hydroxyl group with a fatty acid (partial structure: -OCOR in which R may be any hydrocarbon type functional group). More specifically, the derivative having a fatty acid ester group of a penta-cyclic triterpene used in the present invention means a derivative capable of being formed from the hydroxyl group thereof with a fatty acid, among others. In this regard, the fatty acid is not restricted to any particular one, but specific examples thereof include acetic acid, acetic anhydride, propionic acid, butyric acid, isobutyric acid, valeric acid, isovaleric acid, pivalic acid, caproic acid, caprylic acid, capric acid, undecanoic acid, lauric acid, myristic acid, palmitic acid, palmitoleic acid, stearic acid, oleic acid, elaidic acid, vaccenic acid, linoleic acid, lino-elaidic acid, linolenic acid, γ-linolenic acid, arachidic acid, arachidonic acid, eicosapentaenoic acid, behenic acid, docosahexaenoic acid, lignoceric acid, cerotic acid, montanic acid and melissic acid. Among these, preferred are derivatives derived from acetic acid, acetic anhydride, caproic acid, caprylic acid, capric acid, lauric acid, myristic acid, palmitic acid, palmitoleic acid, stearic acid, oleic acid, elaidic acid, linoleic acid, lino-elaidic acid, linolenic acid, γ-linolenic acid, arachidic acid, arachidonic acid, eicosapentaenoic acid, behenic acid and docosahexaenoic acid.

The alkoxy group means a functional group formed as a result of the usual dehydration reaction between a hydroxyl group and an alcohol (partial structure: -OR in which R represents an arbitrary hydrocarbon type functional group). More specifically, the derivative carrying an alkoxy group of a penta-cyclic triterpene used in the present invention means one capable of being formed from the hydroxyl group of the triterpene and an alcohol, among others. The alcohol used in this derivatization is not restricted to any particular one, but specific examples thereof are methanol, ethanol, n-propanol, isopropanol, allyl alcohol, n-butanol, sec-butanol, tert-butanol, ethylene glycol, trimethylsilyl alcohol, triethylsilyl alcohol, phenol, benzyl alcohol and saccharides. Among them, preferred are derivatives derived from ethanol, triethylsilyl alcohol, methanol, n-propanol, isopropanol and trimethylsilyl alcohol.

The alkoxymethyl group means a functional group formed as a result of the usual dehydration reaction between a hydroxymethyl group and an alcohol (partial structure: -CH2OR in which R represents an arbitrary hydrocarbon type functional group). More specifically, the derivative carrying an alkoxymethyl group of a penta-cyclic triterpene used in the present invention means one capable of being formed from the hydroxyl-methyl group of the triterpene and an alcohol, among others. The alcohol used in this derivatization is not restricted to any particular one, but specific examples thereof are methanol, ethanol, n-propanol, isopropanol, allyl alcohol, n-butanol, sec-butanol, tert-butanol, ethylene glycol, trimethylsilyl alcohol, triethylsilyl alcohol, phenol, benzyl alcohol and saccharides. Among them, preferred are derivatives derived from ethanol, triethylsilyl alcohol, methanol, n-propanol, isopropanol and trimethylsilyl alcohol.

Moreover, the glycosides used in the present invention are derivatives, which can be prepared through the reaction of carboxyl groups, hydroxyl groups and/or hydroxymethyl groups of the foregoing alcohol ester group-containing derivatives, alkoxy group-containing derivatives and alkoxymethyl group-containing derivatives among the penta-cyclic triterpenes with saccharides, among others (partial structure: -COOR, -OR, -CH2OR in which R represents an arbitrary saccharide). The saccharides used in the derivatization are not restricted to particular ones, but specific examples thereof include glucose, mannose, galactose, fructose, xylose, arabinose, fucose, rhamnose, glucosamine, galactosamine and glucuronic acid, either of which may be in either α- or β-form. Moreover, these glycosides may be a monosaccharide or an oligosaccharide higher than disaccharide comprising a variety of combinations of monosaccharides. Some of these glycosides exist in nature and are known generically as "saponin". Both of them can be used in the present invention.

The present invention relates to an external agent for the skin and a whitening agent each comprising, as an effective component, maslinic acid, erythrodiol, uvaol, betulinic acid, betulin and a physiologically acceptable salt thereof or a derivative thereof. The phrase "comprising as an effective component" herein used means that these agents contain the foregoing substances in such an amount sufficient for obtaining the desired whitening effect or a melanogenesis-inhibitory function or the like. More specifically, the amount required for achieving the effect means the amount of maslinic acid, erythrodiol, uvaol, betulinic acid, betulin and a physiologically acceptable salt thereof or a derivative thereof to be incorporated into the external agent for the skin and the whitening agent of the present invention, which is required for ensuring the desired whitening effect on the skin.

Regarding maslinic acid and erythrodiol as oleanane-type triterpenes, uvaol as an ursane-type triterpene and betulinic acid and betulin as lupane-type triterpenes as well as physiologically acceptable salts thereof or derivatives thereof, the origins thereof are not limited to specific ones and those usable herein may be naturally occurring ones, those artificially prepared and/or commercially available ones.

Both of maslinic acid and erythrodiol belong to the oleanane-type triterpenes and it has already been known that they are present in a variety of plants. When the external agent for the skin and whitening agent of the present invention comprise maslinic acid, erythrodiol, physiologically acceptable salts thereof and/or derivatives thereof, the origins thereof are not restricted to any particular one and usable herein include, for instance, naturally occurring ones, artificially synthesized ones and/or commercially available ones.

Maslinic acid is one of the oleanane-type triterpenes and a compound having a structure represented by the foregoing structural formula 1. It has been known that this compound possesses, for instance, an anti-inflammatory effect and an anti-histamic effect. It is also known that the compound is present in natural plants such as olive, hop, mint, pomegranate, clove, sage and jujube. In the external agent for the skin and the whitening agent of the present invention, the origins of maslinic acid and physiologically acceptable salts thereof or derivatives thereof are not limited to specific ones and usable herein include, for instance, naturally occurring ones, artificially prepared ones and/or commercially available ones, with those derived from natural substances such as olive, hop, mint, pomegranate, clove, sage and jujube being preferred. In particular, examples of stable supply sources are olive plants. Olive plants are preferred supply sources since they have been grown stably and continuously and they contain maslinic acid and/or physiologically acceptable salts thereof in high concentrations. Preferably used herein as raw materials also include products obtained during the olive oil-production processes.

<structural formula 1>

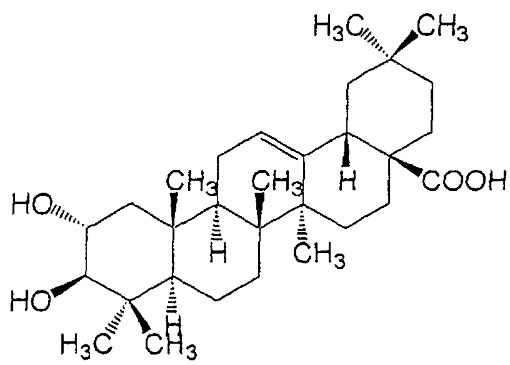

In the present invention, the physiologically acceptable salts and derivatives of maslinic acid are the same as those described above. More specifically, the physiologically acceptable salts are those derived from the group: -COOH shown in the structural formula 1 and are not particularly restricted inasmuch as they are commonly used in foods and beverages as well as pharmaceutical compositions. Specific examples thereof preferably used herein are sodium, potassium, ammonium, dimethylammonium, calcium and magnesium salts for the salt of maslinic acid, with sodium and potassium salts of maslinic acid being preferred.

Examples of derivatives of maslinic acid, which are derivatized only one site, include methyl ester, ethyl ester, n-propyl ester, isopropyl ester, n-butyl ester, trimethylsilyl ester, triethylsilyl ester, β-D-glucopyranosyl ester and β-D-galactopyranosyl ester of maslinic acid, 3-O-acetyl-maslinic acid, 3-O-propionyl-maslinic acid, 3-O-butyryl-maslinic acid, 3-O-valeryl-maslinic acid, 3-O-capryl-maslinic acid, 3-O-lauryl-maslinic acid, 3-O-myristyl-maslinic acid, 3-O-palmityl-maslinic acid, 3-O-palmitoleyl-maslinic acid, 3-O-stearoyl-maslinic acid, 3-O-oleyl-maslinic acid, 3-O-vaccenyl-maslinic acid, 3-O-linoleyl-maslinic acid, 3-O-linolenyl-maslinic acid, 3-O-arachidyl-maslinic acid, 3-O-arachidonyl-maslinic acid, 3-O-behenyl-maslinic acid, 2-O-acetyl-maslinic acid, 2-O-propionyl-maslinic acid, 2-O-butyryl-maslinic acid, 2-O-valeryl-maslinic acid, 2-O-capryl-maslinic acid, 2-O-lauryl-maslinic acid, 2-O-myristyl-maslinic acid, 2-O-palmityl-maslinic acid, 2-O-palmitoleyl-maslinic acid, 2-O-stearoyl-maslinic acid, 2-O-oleyl-maslinic acid, 2-O-vaccenyl-maslinic acid, 2-O-linoleyl-maslinic acid, 2-O-linolenyl-maslinic acid, 2-O-arachidyl-maslinic acid, 2-O-arachidonyl-maslinic acid, 2-O-behenyl-maslinic acid, 3-O-methyl-maslinic acid, 3-O-ethyl-maslinic acid, 3-O-t-butyl-maslinic acid, 3-O-trimethylsilyl-maslinic acid, 3-O-triethylsilyl-maslinic acid, 3-O-benzyl-maslinic acid, 3-O- β -D-glucopyranosyl-maslinic acid, 3-O- β -D-galactopyranosyl-maslinic acid, 3-O- β -D-glucuronopyranosyl- maslinic acid, 2-O-methyl-maslinic acid, 2-O-ethyl-maslinic acid, 2-O-t-butyl-maslinic acid, 2-O-trimethylsilyl-maslinic acid, 2-O-triethylsilyl-maslinic acid, 2-O-benzyl- maslinic acid, 2-O- β -D-glucopyranosyl- maslinic acid, 2-O-β-D-galactopyranosyl- maslinic acid and 2-O- β -D-glucuronopyranosyl- maslinic acid. Among these derivatives, preferred are ethyl ester and triethylsilyl ester of maslinic acid, 3-O-acetyl-maslinic acid, 2-O-acetyl-maslinic acid, 2-O-triethylsilyl-maslinic acid, 3-O-stearoyl-maslinic acid and 2-O-stearoyl-maslinic acid. The foregoing are derivatives in which only one site or group is used for the derivatization, but derivatives may be those derivatized at two or more sites. Examples of preferred such derivatives are 2,3-O-diacetyl derivatives, 2,3-O-ditriethylsilyl derivatives and 2,3-O-distearoyl derivatives of maslinic acid or the foregoing preferred esters thereof. In addition, only monosaccharides are listed above as the glycosides, but they may be oligosaccharides comprising a variety of combinations of disaccharides or higher saccharides.

Erythrodiol is one of the oleanane-type triterpenes, has a structure represented by the structural formula 2 and it has been known that this compound shows, for instance, an anti-inflammatory effect (Planta. Med., 1995, Vol. 61, No. 2, pp. 182-185). There has been known that this compound is present in nature and examples of natural sources thereof include olive, sunflower, common marigold, gum Senegal and red sanders. In the external agent for the skin and the whitening agent according to the present invention, the origins of erythrodiol or derivatives thereof are not restricted to particular ones, usable herein include naturally occurring ones, artificially synthesized ones and commercially available ones. For instance, preferably used herein are those prepared from natural substances such as olive, sunflower, common marigold, gum Senegal and red sanders. In particular, olive is preferred as a supply source thereof and more specifically, preferred are those obtained from olive plants and/or products obtained during the olive oil production processes.

<structural formula 2>

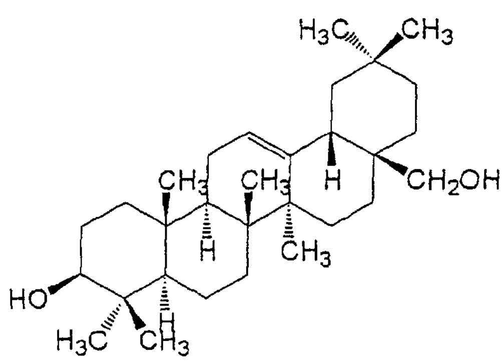

The physiologically acceptable salts and derivatives of erythrodiol are the same as those discussed above.

Examples of derivatives of erythrodiol are those listed below (derivatized only at one site or group), but the present invention is not restricted to these examples at all: 3-O-acetyl-erythrodiol, 3-O-propionyl-erythrodiol, 3-O-butyryl-erythrodiol, 3-O-valeryl-erythrodiol, 3-O-capryl-erythrodiol, 3-O-lauryl-erythrodiol, 3-O-myristyl-erythrodiol, 3-O-palmityl-erythrodiol, 3-O-palmitoleyl-erythrodiol, 3-O-stearoyl-erythrodiol, 3-O-oleyl-erythrodiol, 3-O-vaccenyl-erythrodiol, 3-O-linoleyl-erythrodiol, 3-O-linolenyl-erythrodiol, 3-O-arachidyl-erythrodiol, 3-O-arachidonyl-erythrodiol, 3-O-behenyl-erythrodiol, 28-O-acetyl-erythrodiol, 28-O-propionyl-erythrodiol, 28-O-butyryl-erythrodiol, 28-O-valeryl-erythrodiol, 28-O-capryl-erythrodiol, 28-O-lauryl-erythrodiol, 28-O-myristyl-erythrodiol, 28-O-palmityl-erythrodiol, 28-O-palmitoleyl-erythrodiol, 28-O-stearoyl-erythrodiol, 28-O-oleyl-erythrodiol, 28-O-vaccenyl-erythrodiol, 28-O-linoleyl-erythrodiol, 28-O-linolenyl-erythrodiol, 28-O-arachidyl-erythrodiol, 28-O-arachidonyl-erythrodiol, 28-O-behenyl- erythrodiol, 3-O-methyl-erythrodiol, 3-O-ethyl-erythrodiol, 3-O-t-butyl-erythrodiol, 3-O-trimethylsilyl-erythrodiol, 3-O-triethylsilyl-erythrodiol, 3-O-benzyl-erythrodiol, 28-O-methyl-erythrodiol, 28-O-ethyl-erythrodiol, 28-O-t-butyl-erythrodiol, 28-O-trimethylsilyl-erythrodiol, 28-O-triethylsilyl-erythrodiol, 28-O-benzyl-erythrodiol, 3-O-β-D-glucopyranosyl-erythrodiol, 3-O- β -D-galactopyranosyl-erythrodiol, 3-O- β -D-glucuronopyranosyl-erythrodiol, 28-O-β-D-glucopyranosyl-erythrodiol, 28-O- β -D-galactopyranosyl-erythrodiol and 28-O- β -D-glucuronopyranosyl-erythrodiol. Among these, preferred are 3-O-acetyl-erythrodiol and 28-O-acetyl-erythrodiol. The foregoing are derivatives in which only one site or group is used for the derivatization, but derivatives may be those derivatized at two or more possible sites. An example of such derivative is 3,28-O-diacetyl-erythrodiol. In addition, only monosaccharides are listed above as the glycosides of

erythrodiol, but they may be oligosaccharides comprising a variety of combinations of disaccharides or higher saccharides.

Uvaol is a kind of the ursane-type triterpenes and it is known that this compound is present in a variety of plants. In addition, the physiologically acceptable salts and derivatives thereof are the same as those discussed above. When the external agent for the skin and the whitening agent of the present invention each comprises uvaol, a physiologically acceptable salt thereof or a derivative thereof, the origins of these substances are not restricted to specific ones and usable herein include naturally occurring ones, artificially prepared ones and/or commercially available ones, with the use of naturally occurring ones being preferred.

Uvaol is a kind of ursane-type triterpenes and has a structure represented by the structural formula 3 and the compound has been known to have, for instance, an anti-inflammatory effect (Planta. Med., 1995, Vol. 61, No. 2, pp. 182-185) and a glycerophosphoric acid dehydrogenase-inhibitory function (J.P. KOKAI No. Hei 9-67249). It is known that the compound is present in nature and exists in, for instance, olive, bearberry, sage, gum Senegal and cajeput tree. In the external agent for the skin and the whitening agent according to the present invention, the origins of uvaol or derivatives thereof are not restricted to particular ones, usable herein include naturally occurring ones, artificially synthesized ones and commercially available ones. For instance, preferably used herein are those prepared from natural substances such as olive, bearberry, sage, gum Senegal and cajeput tree. In particular, olive is preferred as a supply source thereof and more specifically, preferred are those obtained from olive plants and/or products obtained during the olive oil production processes.

<structural formula 3>

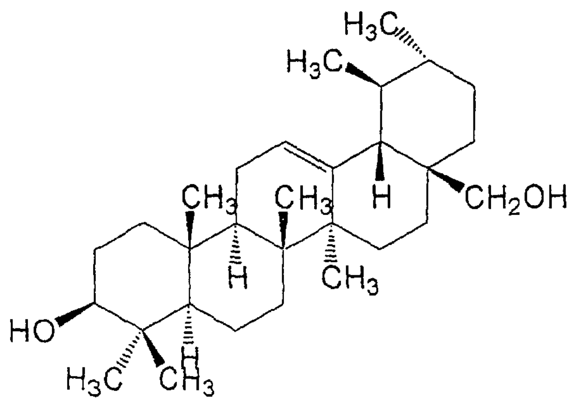

The physiologically acceptable salts and derivatives of uvaol are the same as those discussed above.

Examples of derivatives of uvaol are those listed below (derivatized only at one site or group), but the present invention is not restricted to these examples at all: 3-O-acetyl-uvaol, 3-O-propionyl-uvaol, 3-O-butyryl-uvaol, 3-O-valeryl-uvaol, 3-O-capryl-uvaol, 3-O-lauryl-uvaol, 3-O-myristyl-uvaol, 3-O-palmityl-uvaol, 3-0-palmitoleyl-uvaol, 3-O-stearoyl-uvaol, 3-O-oleyl-uvaol, 3-O-vaccenyl-uvaol, 3-O-linoleyl-uvaol, 3-O-linolenyl-uvaol, 3-O-arachidyl-uvaol, 3-O-arachidonyl-uvaol, 3-O-behenyl-uvaol, 28-O-acetyl-uvaol, 28-O-propionyl-uvaol, 28-O-butyryl-uvaol, 28-O-valeryl-uvaol, 28-O-capryl-uvaol, 28-O-lauryl-uvaol, 28-O-myristyl-uvaol, 28-O-palmityl-uvaol, 28-O-palmitoleyl-uvaol, 28-O-stearoyl-uvaol, 28-O-oleyl-uvaol, 28-O-vaccenyl-uvaol, 28-O-linoleyl-uvaol, 28-O-linolenyl-uvaol, 28-O-arachidyl-uvaol, 28-O-arachidonyl-uvaol, 28-O-behenyl-uvaol, 3-O-methyl-uvaol, 3-O-ethyl-uvaol 3-O-t-butyl-uvaol, 3-O-trimethylsilyl-uvaol, 3-O-triethylsilyl-uvaol, 3-O-benzyl-uvaol, 28-O-methyl-uvaol, 28-O-ethyl-uvaol, 28-O-t-butyl-uvaol, 28-O-trimethylsilyl-uvaol, 28-O-triethylsilyl-uvaol, 28-O-benzyl-uvaol, 3-O-β-D-glucopyranosyl-uvaol, 3-O-β-D-galactopyranosyl-uvaol, 3-O-β-D-glucuronopyranosyl-uvaol, 28-O-β-D-glucopyranosyl-uvaol, 28-O-β-D-galactopyranosyl-uvaol and 28-O-β-D-glucuronopyranosyl-uvaol. Among these, preferred are 3-O-acetyl-uvaol and 28-O-acetyl-uvaol. The foregoing are derivatives in which only one site or group is used for the derivatization, but derivatives may be those derivatized at two or more possible sites. An example of such derivative is 3,28-O-diacetyl-uvaol. In addition, only monosaccharides are listed above as the glycosides of uvaol, but they may be oligosaccharides comprising a variety of combinations of disaccharides or higher saccharides.

Both of betulinic acid and betulin belong to the lupane-type triterpenes and it is known that they are substances existing in a variety of plants. In addition, the physiologically acceptable salts and derivatives thereof are the same as those discussed above. When the external agent for the skin and the whitening agent of the present invention each comprises betulinic acid, betulin, a physiologically acceptable salt thereof or a derivative thereof, the origins of these

substances are not restricted to specific ones and usable herein include naturally occurring ones, artificially prepared ones and/or commercially available ones, with the use of naturally occurring ones being preferred.

Betulinic acid is a kind of lupane-type triterpenes and have a structure represented by the structural formula 4 and it is known that the compound shows a variety of functions such as a cancer-inhibitory function, an anti-inflammatory function, a wound-healing promoting function (J.P. KOKOKU No. Hei 4-26623), an alcohol absorption-inhibitory function (J.P. KOKAI No. Hei 7-53385) and a hair growth-promoting function (J.P. KOKAI No. Hei 9-157139). It is also known that this compound is present in nature and examples of natural sources thereof are gentiana japonica Shult, clove, the rind of grape and olive, in which it is present in the free state, and Panacls Japonici Rhizoma, carrot and sugar beet wherein it is present in the form of saponin. In the external agent for the skin and the whitening agent according to the present invention, the origins of betulinic acid, physiologically acceptable salts thereof or derivatives thereof are not restricted to particular ones, usable herein include naturally occurring ones, artificially synthesized ones and commercially available ones. For instance, preferably used herein are those prepared from natural substances such as gentiana japonica Shult, clove, grape, olive, Panacls Japonici Rhizoma, carrot and sugar beet. In particular, olive is preferred as a supply source thereof and more specifically, preferred are those obtained from olive plants and/or products obtained during the olive oil production processes.

<structural formula 4>

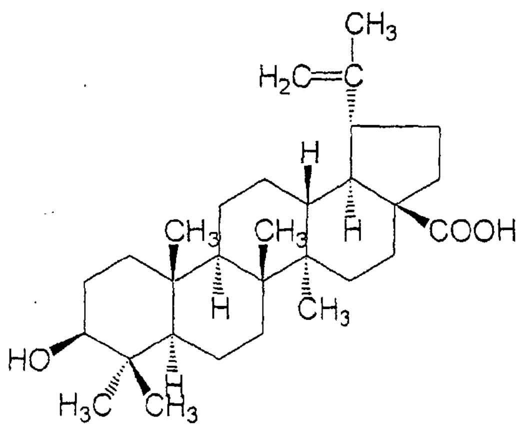

The physiologically acceptable salts and derivatives of betulinic acid are the same as those discussed above.

In this respect, the physiologically acceptable salts of betulinic acid are not restricted to specific ones, but specific examples thereof are sodium, potassium, ammonium, dimethylammonium, calcium and magnesium salts thereof, with sodium and potassium salts of betulinic acid being preferred.

Specific examples of derivatives of betulinic acid (derivatized only at one site or group) include methyl ester, ethyl ester, n-propyl ester, isopropyl ester, n-butyl ester, trimethylsilyl ester and triethylsilyl ester of betulinic acid, betulinic acid- β-D-glucopyranosyl ester, betulinic acid- β -D-galactopyranosyl ester, 3-O-acetyl-beturinic acid, 3-O-propionyl-betulinic acid, 3-O-butyryl-betulinic acid, 3-O-valeryl-betulinic acid, 3-O-capryl-betulinic acid, 3-O-lauryl-betulinic acid, 3-O-myristyl-betulinic acid, 3-O-palmityl-betulinic acid, 3-O-palmitoleyl-betulinic acid, 3-O-stearoyl-betulinic acid, 3-O-oleyl-betulinic acid, 3-O-vaccenyl-betulinic acid, 3-O-linoleyl-betulinic acid, 3-O-linolenyl-betulinic acid, 3-O-arachidyl-betulinic acid, 3-O-arachidonyl-betulinic acid, 3-O-behenyl-betulinic acid, 3-O-methyl-betulinic acid, 3-O-ethyl-betulinic acid, 3-O-t-butyl-betulinic acid, 3-O-trimethylsilyl-betulinic acid, 3-O-triethylsilyl-betulinic acid, 3-O-benzyl-betulinic acid, 3-O- β -D-glucopyranosyl-betulinic acid, 3-O-β-D-galacto-pyranosyl-betulinic acid and 3-O- β -D-glucuronopyranosyl-betulinic acid. Preferred is ethyl ester of betulinic acid, among others. The foregoing are derivatives in which only one site or group is used for the derivatization, but derivatives may be those derivatized at two or more possible sites. In addition, only monosaccharides are listed above as the glycosides of uvaol, but they may be oligosaccharides comprising a variety of combinations of disaccharides or higher saccharides.

Betulin is a kind of lupane-type triterpenes and has a structure represented by the structural formula 5 and it has been known that the compound has a variety of functions such as a biological protein modification-inhibitory function (J.P. KOKAI No. Hei 9-67253), a glycerophosphoric acid dehydrogenase-inhibitory function (J.P. KOKAI No. Hei 9-67249), a lipase-inhibitory function (J.P. KOKAI No. Hei 10-265328) and a liver disease-prophylactic function (J.P. KOKAI No. Hei 11-209275). It is known that the compound exists in nature, for instance, in the bark of white birch. In

the external agent for the skin and the whitening agent of the present invention, the origins of betulin or derivatives thereof are not restricted to specific ones and usable herein include naturally occurring ones, artificially prepared ones and/or commercially available ones, with the use of those derived from naturally occurring substances such as the bark of white birch being preferred.

<structural formula 5>

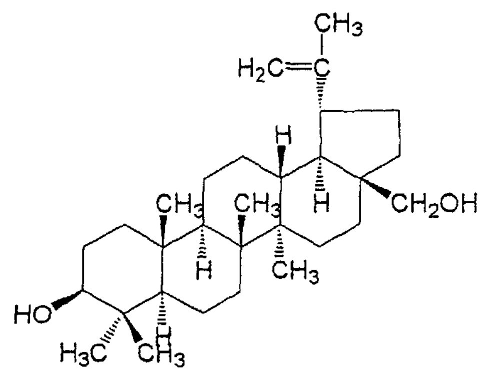

The physiologically acceptable salts and derivatives of betulin are the same as those discussed above.

In this regard, the derivatives of betulin are not restricted to particular ones, but specific examples thereof (derivatized only at one site or group) include 3-O-acetyl-betulin, 3-O-propionyl-betulin, 3-O-butyryl-betulin, 3-O-valeryl-betulin, 3-O-capryl-betulin, 3-O-lauryl-betulin, 3-O-myristyl-betulin, 3-O-palmityl-betulin, 3-O-palmitoleyl-betulin, 3-O-stearoyl-betulin, 3-O-oleyl-betulin, 3-O-vaccenyl-betulin, 3-O-linoleyl-betulin, 3-O-linolenyl-betulin, 3-O-arachidyl-betulin, 3-O-arachidonyl-betulin, 3-O-behenyl-betulin, 28-O-acetyl-betulin, 28-O-propionyl-betulin, 28-O-butyryl-betulin, 28-O-valeryl-betulin, 28-O-capryl-betulin, 28-O-lauryl-betulin, 28-O-myristyl-betulin, 28-0-palmityl-betulin, 28-O-palmitoleyl-betulin, 28-O-stearoyl-betulin, 28-O-oleyl- betulin, 28-O-vaccenyl-betulin, 28-0-linoleyl-betulin, 28-O-linolenyl-betulin, 28-O- arachidyl-betulin, 28-O-arachidonyl-betulin, 28-O-behenyl-betulin, 3-O-methyl-betulin, 3-O-ethyl-betulin, 3-O-t-butyl-betulin, 3-O-trimethylsilyl-betulin, 3-O-triethylsilyl- betulin, 3-O-benzyl-betulin, 28-O-methyl-betulin, 28-O-ethyl-betulin, 28-O-t-butyl- betulin, 28-O-trimethylsilyl-betulin, 28-O-triethylsilyl-betulin, 28-O-benzyl-betulin, 3-O- β -D-glucopyranosyl-betulin, 3-O- β-D-galactopyranosyl-betulin, 3-O- β -D-glucuronopyranosyl-betulin, 28-O-β-D-glucopyranosyl-betulin, 28-O-β-D-galacto-pyranosyl-betulin and 28-O-β-D-glucuronopyranosyl-betulin. Among these, preferred are 3-O-acetyl-betulin and 28-O-acetyl-betulin. The foregoing are derivatives in which only one site or group is used for the derivatization, but derivatives may naturally be those derivatized at two or more possible sites. An example of such derivative is 3,28-O-diacetyl-betulin. In addition, only monosaccharides are listed above as the glycosides of betulin, but they may be oligosaccharides comprising a variety of combinations of disaccharides or higher saccharides.

As has been described above, the inventors of this invention have found that maslinic acid, erythrodiol, uvaol, betulinic acid, betulin and/or physiologically acceptable salts thereof or derivatives thereof possess whitening effects and, in particular, maslinic acid and/or physiologically acceptable salts thereof or derivatives thereof show considerably excellent whitening effects.

In the present invention, the term "whitening effect" means the effect of relieving or lightening and/or preventing the dark skin, melasma, ephelis and darkening or dullness of the skin caused due to a variety of causes such as the exposure of the skin to UV light rays, the paraeccrisis of hormones and genetic programs. In general, it has been known that melanocytes are stimulated by, for instance, the exposure of the skin to UV light rays and any change in the balance between hormones and the melanin thus biosynthesized in the melanocytes precipitates within the skin to thus cause the dark skin, melasma, ephelis and darkening or dullness of the skin. For this reason, the control of the foregoing melanogenesis would permit the relief, lightening and/or prevention of any occurrence of the dark skin, melasma, ephelis and darkening or dullness of the skin.

In the present invention, the whitening effect means, for instance, an effect of making the skin beautiful and/or rich in transparency; an effect of maintaining the skin in its transparent and beautiful conditions; an effect of relieving the darkening or dullness of the skin and of increasing the tenseness of the skin; and an effect of preventing or relieving and/or lightening the dark skin, melasma and ephelis of the skin due to, for instance, suntan.

With regard to the evaluation of the whitening effect in the present invention, the primary evaluation thereof was conducted by carrying out cell-whitening tests (tests for evaluating the melanogenesis-inhibitory function) and tests for evaluating cytotoxicity using experimental systems of B-16 melanoma cell-cultivation to thus evaluate the effect and compare the results obtained with one another. In addition, the whitening effect of each substance was inspected for the practical effectiveness using practically prepared cosmetics using the substances, as the secondary evaluation. The penta-cyclic triterpenes whose strong melanogenesis-inhibitory effect had been elucidated in the foregoing cellular evaluation as the primary evaluation showed an excellent effect even in the secondary evaluation. Moreover, there was observed a correlation between the effects obtained in these two evaluation methods.

The B-16 melanoma cell is one having an ability of producing melanin and therefore, the cells naturally grown by cell culture blacken. If a substance capable of inhibiting melanogenesis is present in the cell growth system during the cell growth, the blackening of the cells is inhibited and they relatively whiten. Accordingly, the whitening effect of each candidate substance can be evaluated on the basis of the degree of whiteness observed when adding a test sample relative to the degree of blackness observed for a control. Moreover, some of test substances possess toxicity to B-16 melanoma cells. To evaluate this cytotoxicity, the cell survival rate observed when adding a test sample to the system is compared with that observed for the control. Thus, the substantial whitening effect can be evaluated on the basis of the results of these cell-whitening test and cytotoxicity test.

An example of such a method for evaluating the melanogenesis-inhibitory function in a cell culture system using B-16 melanoma cells comprises, for instance, the steps of dispensing 2 ml/well of a culture medium to each well of a 6-well plate, inoculating a desired amount of melanoma cells to the medium, cultivating the cells by allowing the medium to stand at 37°C and a carbon dioxide concentration of 5%. On the following day, a solution of a test sample is added and mixed with the culture medium to a predetermined concentration and the cell cultivation is continued. After 5 days from the initiation of the cultivation, the culture medium is replaced with fresh one and the solution of the test sample is again added thereto. On the next day, the medium is removed to thus recover the cells, followed by washing them with PBS (phosphate-buffered saline) and the determination of the degree of whiteness to thus evaluate the whitening effect of the test sample. In this connection, the melanogenesis-inhibitory function is evaluated by comparing the results thus obtained with the degree of whiteness observed when the same procedures are repeated using the vitamin C-magnesium phosphate as a known whitening agent instead of the test sample solution (positive control) and that observed when the procedures are repeated without addition of any test sample (control).

As a result of the primary evaluation or the whitening effect-evaluation test using cell cultivation systems, it was found that maslinic acid, erythrodiol, uvaol, betulinic acid, betulin and/or physiologically acceptable salts thereof or derivatives thereof had quite strong whitening effects (or high melanogenesis-inhibitory function). For instance, the whitening effects were found to be about 100 to 200 times for maslinic acid, about 100 to 200 times for a salt thereof, about 10 to 100 times for erythrodiol, about 30 to 120 times for uvaol, about 30 to 120 times for betulinic acid, about 10 to 100 times for betulin, about 100 to 200 times for ethyl ester of maslinic acid, about 50 to 150 times for acetylated maslinic acid, about 30 to 120 times for triethyl-silylated maslinic acid, about 50 to 150 times for ethyl ester of stearoyl-modified maslinic acid, about 10 to 100 times for acetylated erythrodiol, about 10 to 100 times for acetylated uvaol, about 10 to 100 times for ethyl ester of betulinic acid and about 5 to 50 times for acetylated betulin as compared with that observed for the control. In other words, the external agent for the skin and the whitening agent of the present invention show quite strong melanogenesis-inhibitory function since they contain penta-cyclic triterpenes and physiologically acceptable salts thereof or derivatives thereof

When controlling the whitening effect to a predetermined level, the resulting whitening agent is inferior in the cytotoxicity as compared with another whitening agent showing a whitening effect almost identical to that of the former while taking into consideration the balance between the whitening effect and the cytotoxicity and therefore, the substances of the present invention possess whitening effects and safety for the skin. For instance, when comparing products comprising the substances of the present invention with those comprising, for instance, arbutin in a concentration for ensuring a whitening effect almost identical to that of the former, maslinic acid, erythrodiol, uvaol, betulinic acid, betulin and/or physiologically acceptable salts thereof or derivatives thereof have low cytotoxicity and therefore, the incorporation of these substances of the present invention would permit the preparation of an external agent for the skin and a whitening agent having high safety for the skin. In this respect, however, the incorporation thereof in excess may adversely affect the skin and accordingly, it is necessary to incorporate these substances into these agents in an amount suitable for achieving the desired whitening effect.

The external agent for the skin of the present invention possesses a quite excellent whitening effect.

As has been described above, if an agent shows not only a whitening effect, but also an effect of inhibiting the melanogenesis, the agent would permit the prevention and/or relief or lightening of the dark skin, melasma, ephelis and darkening or dullness of the skin. In this respect, the external agent for the skin according to the present invention would permit the control of the melanogenesis to a level as low as possible since maslinic acid, erythrodiol, uvaol, betulinic acid, betulin and/or physiologically acceptable salts thereof or derivatives thereof used in the agent have strong melanogenesis-inhibitory effects. Therefore, the external agent for the skin permits the prevention and/or relief

or lightening of the dark skin, melasma, ephelis and darkening or dullness of the skin and shows an excellent whitening effect.

In the secondary evaluation of the whitening effect of the external agent for the skin, the effectiveness thereof can be confirmed by the evaluation of the effect using practical cosmetics prepared. For instance, the effectiveness thereof can be evaluated by preparing a variety of external agents for the skin or the like such as a cream, a toilet water and a milky lotion, which comprise at least one member selected from the group consisting of maslinic acid, erythrodiol, uvaol, betulinic acid, betulin and/or physiologically acceptable salts thereof or derivatives thereof according to the present invention and carrying out sensory tests using the resulting test external agent or the like and female panelists. More specifically, a test external agent for the skin thus prepared is applied to the skin of 20 to 50-year-old female panelists (15), twice a day (in the morning and night) over a plurality of weeks. Thus, the whitening effect through the application of the agent can be evaluated according to the following 3-stage criteria: effective, slightly effective and ineffective (or not effective) for making the melasma, ephelis and darkening or dullness of the skin inconspicuous.

In the evaluation procedures, the external agent for the skin or the like of the present invention shows a whitening effect identical or superior to those observed for agents comprising, for instance, arbutin and kojic acid having excellent whitening effects as controls. In other words, the external agent for the skin of the invention possesses an excellent whitening effect.

Since maslinic acid, erythrodiol, uvaol, betulinic acid and betulin are oil-soluble, they can suitably be incorporated into, for instance, emulsion type external agents and whitening agents. In addition, they are preferably used, in particular, in cosmetics, since they have a good absorbency through the skin because of their good oil-solubility and they can suitably exert a whitening effect on the skin.

Moreover, physiologically acceptable salts or derivatives of maslinic acid, erythrodiol, uvaol, betulinic acid and betulin may be water-soluble depending on the kinds of the salts or derivatives and therefore, they can suitably and uniformly be dissolved or dispersed in, for instance, aqueous or emulsion type external agents for the skin and whitening agents. A large number of cosmetics are commercialized in the form of aqueous or emulsified forms. In this case, maslinic acid, erythrodiol, uvaol, betulinic acid and betulin may, if necessary, be converted into physiologically acceptable salts thereof before they are incorporated into these cosmetic products.

Moreover, maslinic acid, erythrodiol, uvaol, betulinic acid, betulin and/or physiologically acceptable salts thereof or derivatives thereof permit the achievement of, for instance, such a whitening effect on the order of the currently required level by the incorporation thereof into an external agent for the skin and a whitening agent in a quite small amount. For this reason, the compounds have a merit in cost, they can provide room for the incorporation of other components into the agents, while taking into consideration the mixing ratio and therefore, the functions of the resulting agents may further be enriched.

Naturally, the present invention permits the preparation of an external agent for the skin and a whitening agent having significantly high whitening effects by increasing the amount, to be incorporated, of maslinic acid, erythrodiol, uvaol, betulinic acid, betulin and/or physiologically acceptable salts thereof or derivatives thereof.

The present invention relates to a novel external agent for the skin comprising at least one member selected from the group consisting of maslinic acid, erythrodiol, uvaol, betulinic acid, betulin and/or physiologically acceptable salts thereof or derivatives thereof and preferably to an external agent for the skin comprising at least one member selected from the group consisting of maslinic acid, erythrodiol, uvaol, betulinic acid, betulin and/or physiologically acceptable salts thereof or derivatives thereof, as a whitening component.

The term "external agent for the skin" herein used includes pharmaceutical agents, quasi-drugs and cosmetics and is an agent applied to the skin. The dosage forms thereof are not restricted to particular ones, but specific examples thereof include skin care cosmetics such as milky lotions, creams, toilet waters, pack and cosmetics for washing or cleansing; make-up cosmetics such as lip colors and foundations; cosmetics for the scalp; and pharmaceutical agents such as ointments, dispersions, creams and liquids for external use.

The content of maslinic acid, erythrodiol, uvaol, betulinic acid, betulin and/or physiologically acceptable salts thereof or derivatives thereof used in the external agent for the skin is not restricted to any specific one, but an external agent for the skin showing an excellent whitening effect and having high safety to the skin can be obtained when using them in an amount preferably ranging from 0.00001 to 10% by mass, more preferably 0.00001 to 5% by mass, more preferably 0.00001 to 3% by mass, more preferably 0.00001 to 5% by mass, more preferably 0.0001 to 5% by mass, more preferably 0.001 to 5% by mass, more preferably 0.01 to 5% by mass, more preferably 0.1 to 5% by mass, more preferably 0.1 to 5% by mass, more preferably 0.5 to 5% by mass, more preferably 1 to 5% by mass, and more preferably 1 to 3% by mass.

When using maslinic acid and/or physiologically acceptable salts or derivatives thereof as the effective components of the external agent for the skin according to the present invention, the agent preferably comprises these components in an amount ranging from 0.00001 to 10% by mass, more preferably 0.0001 to 5% by mass and further preferably 0.001 to 5% by mass.

When erythrodiol and/or physiologically acceptable salts or derivatives thereof are incorporated into the external agent for the skin according to the present invention as effective components, the content thereof preferably ranges from 0.00005 to 10% by mass, more preferably 0.0005 to 6% by mass and further preferably 0.005 to 6% by mass.

When uvaol and/or physiologically acceptable salts or derivatives thereof are incorporated into the external agent for the skin according to the present invention as effective components, the content thereof preferably ranges from 0.00002 to 10% by mass, more preferably 0.0002 to 5.2% by mass and further preferably 0.002 to 5.2% by mass.

When betulinic acid and/or physiologically acceptable salts or derivatives thereof are incorporated into the external agent for the skin according to the present invention as effective components, the content thereof preferably ranges from 0.00002 to 10% by mass, more preferably 0.0002 to 5.2% by mass and further preferably 0.002 to 5.2% by mass.

When betulin and/or physiologically acceptable salts or derivatives thereof are incorporated into the external agent for the skin according to the present invention as effective components, the content thereof preferably ranges from 0.00003 to 10% by mass, more preferably 0.0003 to 5.5% by mass and further preferably 0.003 to 5.5% by mass.

In addition, the present invention also relates to a whitening agent comprising, as an effective component, at least one member selected from the group consisting of maslinic acid, erythrodiol, uvaol, betulinic acid, betulin and/or physiologically acceptable salts or derivatives thereof. As has been described above, maslinic acid, erythrodiol, uvaol, betulinic acid, betulin and/or physiologically acceptable salts or derivatives thereof show quite excellent whitening effects and therefore, the whitening agent of the present invention possesses quite excellent whitening effect.

The phrase "comprising as an effective component" used herein means that the agent comprises these substances in an amount sufficient for achieving the intended whitening effect and the content thereof is not restricted to a specific range, but the whitening agent of the invention comprises maslinic acid, erythrodiol, uvaol, betulinic acid, betulin and/or physiologically acceptable salts or derivatives thereof in an amount of preferably not less than 0.001% by mass, more preferably 0.001 to 99.99% by mass, further preferably 0.05 to 99.9% by mass, further preferably 0.1 to 99.5% by mass, further preferably 0.05 to 99.99% by mass, further preferably 0.1 to 99.99% by mass, further preferably 1 to 99% by mass, further preferably 3 to 99.99% by mass, further preferably 5 to 99.99% by mass, and further preferably 10 to 99,99% by mass on the basis of the total mass of the whitening agent. These substances should preferably used in an amount falling within the range specified above since they favorably show their whitening effects in themselves or even when they are incorporated into, for instance, the external agent for the skin of the present invention.

When the whitening agent comprises, as effective components, maslinic acid and/or physiologically acceptable salts or derivatives thereof, the content thereof is preferably not less than 0.001% by mass, more preferably 0.001 to 99.99% by mass, further preferably 0.05 to 99.99% by mass, further preferably 0.1 to 99.99% by mass and further preferably 1 to 99.99% by mass.

When the whitening agent comprises, as effective components, erythrodiol and/or physiologically acceptable salts or derivatives thereof, the content thereof is preferably not less than 0.005% by mass, more preferably 0.005 to 99.99% by mass, further preferably 0.1 to 99.99% by mass, further preferably 0.2 to 99.99% by mass, further preferably 2.5 to 99.99% by mass, further preferably 4.5 to 99.99% by mass, further preferably 6.5 to 99.99% by mass and further preferably 10 to 99.99% by mass.

When the whitening agent comprises, as effective components, uvaol and/or physiologically acceptable salts or derivatives thereof, the content thereof is preferably not less than 0.002% by mass, more preferably 0.002 to 99.99% by mass, further preferably 0.06 to 99.99% by mass, further preferably 0.12 to 99.99% by mass, further preferably 0.15 to 99.99% by mass, further preferably 3.5 to 99.99% by mass, further preferably 5.5 to 99.99% by mass and further preferably 10 to 99.99% by mass.

When the whitening agent comprises, as effective components, betulinic acid and/or physiologically acceptable salts or derivatives thereof, the content thereof is preferably not less than 0.002% by mass, more preferably 0.02 to 99.99% by mass, further preferably 0.06 to 99.99% by mass, further preferably 0.12 to 99.99% by mass, further preferably 1.5 to 99.99% by mass, further preferably 3.5 to 99.99% by mass, further preferably 5.5 to 99.99% by mass and further preferably 10 to 99.99% by mass.

When the whitening agent comprises, as effective components, betulin and/or physiologically acceptable salts or derivatives thereof, the content thereof is preferably not less than 0.003% by mass, more preferably 0.003 to 99.99% by mass, further preferably 0.07 to 99.99% by mass, further preferably 0.15 to 99.99% by mass, further preferably 2 to 99.99% by mass, further preferably 4 to 99.99% by mass, further preferably 6 to 99.99% by mass and further preferably 10 to 99.99% by mass.

In addition, the whitening agent can be used for the preparation of an external agent for the skin having a whitening effect. The content of the whitening agent in the external agent for the skin is not restricted to any particular range and it may arbitrarily be controlled depending on the desired intensity of such a whitening effect as in the foregoing.

When using maslinic acid, erythrodiol, uvaol, betulinic acid, betulin and/or physiologically acceptable salts or

derivatives thereof as a raw material for a whitening agent, the content thereof in the whitening agent is preferably not less than 0.1% by mass, more preferably 0.1 to 99.99% by mass, further preferably 1 to 99.99% by mass, further preferably 10 to 99.99% by mass, further preferably 30 to 99.99% by mass, further preferably 50 to 99.99% by mass, further preferably 70 to 99.99% by mass, further preferably 80 to 99.99% by mass and further preferably 90 to 99.99% by mass.

Maslinic acid, erythrodiol, uvaol, betulinic acid, betulin and/or physiologically acceptable salts or derivatives thereof can significantly inhibit the production of melanin as a dye. For this reason, the whitening agent comprising at least one member selected from the group consisting of the foregoing substances is provided for the purpose of relief or prevention of, for instance, the dark skin, melasma and ephelis and the purpose can be accomplished by directly applying the agent to the skin. Moreover, the whitening agent can likewise be used for whitening, for instance, the melasma of the skin caused due to burn or the like and the whitening agent may likewise be incorporated into or used as, for instance, an external agent for the skin.

These penta-cyclic triterpenes, as natural substances, can be obtained by extracting them from the corresponding plant's bodies listed above. More specifically, they can be extracted from the corresponding plant's bodies with water and/or organic solvents and the resulting extracts can further be treated by, for instance, solvent extraction methods, methods which make use of the difference in solubility between the triterpenes and impurities, fractional precipitation methods, recrystallization methods, ion-exchange resin methods and liquid chromatography methods, which may be used alone or in any combination or which may repeatedly be used, to thus isolate and/or purify the desired substances.

In particular, maslinic acid and/or physiologically acceptable salts or derivatives thereof can be extracted from olive plants and the resulting extract can further be treated by, for instance, solvent extraction methods, methods which make use of the difference in solubility between these substances and impurities, fractional precipitation methods, recrystallization methods, ion-exchange resin methods and liquid chromatography methods, which may be used alone or in any combination or which may repeatedly be used, to thus isolate and/or purify these substances.

The olive plant (Olea europaea L.) used in the invention as a raw material may be any one irrespective of the habitats and may thus be those home-grown or Europe growth or may be edible ones or those for the oil expression. The maslinic acid and/or physiologically acceptable salts thereof used in the external agent for the skin and the whitening agent of the present invention can be obtained mainly from the fruits or seeds, or further from the seed coats, leaves, stems and buds of the olive plant as a naturally occurring plant. The foregoing substances may likewise suitably be prepared from the dried products, pulverized products and defatted products of the foregoing raw materials.

Moreover, water is preferably added to the foregoing fruits of the olive plant or the defatted product thereof or the fruits or the defatted product thereof are humidified by a steaming treatment. Thus, they appropriately get swollen and therefore, the extraction efficiency is improved.

In particular, the defatted product of the olive plant is preferably used herein since it includes maslinic acid and/or physiologically acceptable salts thereof to be extracted in high concentrations and it is not necessary to remove the oil fraction from the resulting maslinic acid and/or physiologically acceptable salts thereof.

The defatted product can be prepared from the olive oil-extraction residue generated during edible oil-purification processes or the residue obtained after the extraction with, for instance, hexane as a raw material.

In addition, it is also possible to use a defatted product obtained when the lipid components contained in the olive plant or the defatted product thereof are removed by extraction with at least one member selected from the group consisting of hydrocarbons such as pentane, hexane and heptane, lower alkyl fatty acid esters such as ethyl ester of acetic acid and known water-insoluble organic solvents such as diethyl ether and further the foregoing washing operation is if necessary repeated.

Extraction of olive plants with water and/or an organic solvent permits the preparation of maslinic acid and/or physiologically acceptable salts thereof, which can be incorporated into the external agent for the skin and the whitening agent of the present invention.

The organic solvent used for obtaining, from the olive plants, maslinic acid and/or physiologically acceptable salts thereof, which can be incorporated into the external agent for the skin and the whitening agent of the present invention may be either a hydrophilic organic solvent or a hydrophobic organic solvent, but preferably used herein are hydrophilic organic solvents. Specific examples of hydrophilic organic solvents include alcohols such as methyl alcohol, ethyl alcohol, glycerin, propylene glycol and 1,3-butylene glycol; and other known organic solvents such as acetone, tetrahydrofuran, acetonitrile, 1,4-dioxane, pyridine, dimethylsulfoxide, N,N-dimethyl formamide and acetic acid. Among these, preferred are alcohols such as methyl alcohol, ethyl alcohol, glycerin, propylene glycol and 1,3-butylene glycol, with ethanol being particularly preferred. In addition, specific examples of hydrophobic organic solvents are known organic solvents such as hexane, cyclohexane, carbon tetrachloride, chloroform, dichloromethane, 1,2-dichloroethane, diethyl ether, ethyl acetate, benzene and toluene. These organic solvents may be used alone or in any combination of at least two thereof.

The use of a hydrophilic organic solvent is preferred from the industrial standpoint, for instance, from the

viewpoint of the permeability thereof into the plant's tissues and the extraction efficiency and it is also preferred to use water-containing hydrophilic organic solvents. Specific examples thereof are alcohols such as methyl alcohol, ethyl alcohol, glycerin, propylene glycol and 1,3-butylene glycol, other organic solvents such as acetone, tetrahydrofuran and acetonitrile and these solvents containing water. The maslinic acid and/or physiologically acceptable salts thereof used in the external agent for the skin and the whitening agent of the present invention can be obtained from the olive plants, using at least one member selected from the group consisting of the solvents listed above.

The conditions for the extraction are not restricted to any specific one and the extraction can, for instance, be conducted at a temperature ranging from 5 to 95°C, preferably 10 to 90°C and more preferably 15 to 85°C. Alternatively, the extraction can likewise suitably be conducted at ordinary temperature. There is such a tendency that the higher the extraction temperature, the higher the extraction efficiency. The extraction may suitably be carried out at ordinary pressure, under pressure or a reduced pressure established by, for instance, aspiration. Moreover, the extraction may be carried out by the shaking extraction technique or by the use of an extraction device equipped with, for instance, a stirring machine, in order to improve the extraction efficiency. The extraction time may vary depending on other extraction conditions, but in general ranges from several minutes to several hours. In this respect, the longer the extraction time, the higher the extraction efficiency. However, the extraction time may appropriately be determined or selected depending on the production conditions such as production facilities used and intended yields.

In addition, the amount of the solvent used in the extraction preferably ranges from 1 to 100 times ("mass/mass", those in the following description will be shown in the same way also) and more preferably 1 to 20 times that of the raw material.

In particular, the olive plants are preferably extracted with water, water-containing lower alcohols or anhydrous lower alcohols while taking into consideration, for instance, the safety to the skin.

Moreover, if taking into consideration the yield of the resulting maslinic acid and/or physiologically acceptable salts thereof and the intensity of whitening effect, the olive plant is preferably extracted with water-containing lower alcohols having a lower alcohol content of not less than 10% by mass. The water-containing lower alcohol used herein more preferably has a lower alcohol content ranging from 10 to 95% by mass and the lower alcohol content of the water-containing lower alcohol is most preferably adjusted to the range of from 30 to 95% by mass.

In this respect, examples of alcohols used in the present invention are known solvents, for instance, primary alcohols such as methyl alcohol, ethyl alcohol, 1-propanol and 1-butanol, secondary alcohols such as 2-propanol and 2-butanol, tertiary alcohols such as 2-methyl-2-propanol, and liquid polyhydric alcohols such as ethylene glycol, propylene glycol and 1,3-butylene glycol. These solvents may be used alone or in any combination of at least two of them.

The term "lower alcohol" herein used means a known alcohol having 1 to 4 carbon atoms such as a primary, secondary, tertiary or liquid polyhydric alcohol listed above, which may be used alone or in any combination of at least two of them.

Maslinic acid and/or physiologically acceptable salts thereof according to the present invention can be obtained by the removal of the solvent and water from the extract thus prepared.

The solvent and the water can be removed from the extract according to any known method such as distillation under reduced pressure, drying under reduced pressure or in a vacuum, lyophilization (or freeze-drying) and spray drying.

In this respect, it is a matter of course that the extract may be used without removing any such solvent and water and the conditions of the extract are not restricted to any specific one.

The extract derived from a defatted product is preferred since it is free of any oil-soluble component such as triglyceride, sterol and tocopherol and therefore, the extract never requires any step for the removal of these components and any purification step. In addition, the defatted product comprises the residue obtained after the oil expression and therefore, strained lees obtained after olive oil expression and extracted lees can be used. This method accordingly permits the effective use of olive plants and is quite excellent from the viewpoint of production cost since the method makes use of materials commonly disposed or used as feeds.

Moreover, it is preferred to concentrate maslinic acid and/or physiologically acceptable salts thereof extracted from olive plants prior to use the same in, for instance, the external agent for the skin of the present invention in order to make the most use of the whitening effect thereof.

Conditions for such concentration are not restricted to particular ones and an example of such concentration makes use of the solubility in water. The maslinic acid and/or physiologically acceptable salts thereof included in the external agent for the skin and whitening agent of the present invention have a relatively low polarity and are compounds hardly soluble in water. These characteristic properties would permit the separation of the crude extract derived from olive plants into components, which are not easily dissolved in water and/or those insoluble in water or those, for instance, hardly soluble in water and components easily soluble in water to thus substantially concentrate the crude extract. The components, for instance, hardly soluble in water included in the crude extract derived from olive plants are considerably excellent in the whitening effect as compared with the effect observed for the overall crude extract from olive plants and this clearly indicates that maslinic acid and/or physiologically acceptable salts thereof are in fact

concentrated in the components hardly soluble in water.

The components hardly soluble in water or the like can easily be obtained by adding a crude extract from olive plants to water with stirring and then recovering the precipitated fraction through, for instance, filtration.

Alternatively, maslinic acid and/or physiologically acceptable salts thereof incorporated into, for instance, the external agent for the skin according to the present invention can, if necessary, be concentrated by the liquid-liquid fractionation using the usual combination of solvents. The combination of solvents is not unconditionally determined, but examples thereof are combinations of water and hydrophobic organic solvents and specific examples of such hydrophobic organic solvents include known organic solvents such as hexane, carbon tetrachloride, chloroform, dichloromethane, 1,2-dichloroethane, diethyl ether, ethyl acetate, n-butanol, benzene and toluene.

Maslinic acid and/or physiologically acceptable salts thereof are hardly soluble in water and therefore, the hydrophobic organic solvent phase is separated to thus remove unnecessary water-soluble components. The solvents are removed from the resulting organic solvent phase to thus easily concentrate maslinic acid and/or physiologically acceptable salts thereof included therein.

Furthermore, maslinic acid and/or physiologically acceptable salts thereof included therein included in the external agent for the skin and whitening agent of the present invention are preferably fractionated and purified or isolated from the foregoing extract and/or concentrate. Thus, the extract and/or concentrate can further be concentrated to a level higher than that attained by the foregoing concentration procedures to thus isolate the desired components.

The fractionation and/or purification treatment shows such advantages that the treatment permits the substantial improvement of, for instance, the whitening effect and that it also permits the removal of impurities present in the extract. More specifically, the fractionation and/or purification treatment is preferred since it permits the preparation of maslinic acid and/or physiologically acceptable salts thereof as white crystals and it in turn permits the incorporation of the resulting extract into, for instance, the external agent for the skin without undesirably coloring the agent.

The fractionation and/or purification treatment is not unconditionally determined, but specific examples thereof are recrystallization, fractional precipitation and chromatography techniques. Among the chromatography techniques, the liquid chromatography technique is preferably used in the present invention since this technique permits the efficient fractionation and/or purification without decomposing maslinic acid and/or physiologically acceptable salts thereof used in the external agent for the skin and whitening agent of the present invention. Specific examples of such liquid chromatography techniques are normal phase liquid chromatography, reverse phase liquid chromatography, thin-layer liquid chromatography, paper chromatography and high performance liquid chromatography (HPLC). Any one of these chromatography techniques may be used in the fractionation and/or purification treatment of the extract for obtaining maslinic acid and/or physiologically acceptable salts thereof used in the external agent for the skin and whitening agent of the present invention. Among these, preferred are normal phase liquid chromatography, reverse phase liquid chromatography and high performance liquid chromatography (HPLC) techniques, while taking into consideration, for instance, the resolution, the loading and the number of steps required.

In this connection, the normal phase liquid chromatography means, for instance, the following method. In other words, this method comprises the steps of preparing a column in which the stationary phase comprises, for instance, silica gel and the mobile phase comprises, for instance, a hexane-ethyl acetate mixed liquid or a chloroform-methanol mixed liquid; supplying a crude extract from olive plants or the concentrate thereof to the column at a rate of loading ranging 0.1 to 5% (wt (mass)/v (volume)); and then eluting a desired fraction according to the continuous elution method using a single mobile phase or the gradient elution method in which the polarity of the solvent or mobile phase is stepwise increased.

The reverse phase liquid chromatography technique is, for instance, the following method. In other words, this method comprises the steps of preparing a column in which the stationary phase comprises, for instance, silica coupled with octadecyl silane (ODS) and the mobile phase comprises, for instance, a water-methanol mixed liquid, a water-acetonitrile mixed liquid or a water-acetone mixed liquid; supplying a crude extract from olive plants or the concentrate thereof to the column at a rate of loading ranging from 0.1 to 5% (wt (mass)/v (volume)); and then eluting a desired fraction according to the continuous elution method using a single mobile phase or the gradient elution method in which the polarity of the solvent or mobile phase is stepwise increased.

The high performance liquid chromatography (HPLC) technique is, in principle, identical to the foregoing normal phase liquid chromatography and reverse phase liquid chromatography techniques, but is used for the rapid fractionation-purification treatment at a high resolution.

The use of at least one of the foregoing methods is preferred in the present invention since the use thereof would permit the concentration of maslinic acid and/or physiologically acceptable salts thereof to a higher extent and the preparation of a concentrate free of any impurity.

Moreover, the use of at least one of the foregoing methods permits the control of the purity of maslinic acid and/or physiologically acceptable salts thereof and likewise permits the arbitrary design of, for instance, the intensity of the whitening effect and characteristic properties of the resulting product.

The foregoing concentration treatment may preferably be repeated over a desired time and further different

concentration treatments may be used in combination. Similarly, the fractionation-purification treatment may be repeated over a desired time and different fractionation-purification treatments may be used in combination. Moreover, the extract may be subjected to a concentration treatment and then to a fractionation-purification treatment, or it may be subjected to a fractionation- purification treatment and then to another fractionation-purification treatment, or further it may be subjected to a concentration treatment, a fractionation-purification treatment and a concentration treatment, in this order. Naturally, any combination of these treatments may be used, in addition to the foregoing ones.

Maslinic acid and/or physiologically acceptable salts thereof can suitably be obtained by variously combining the foregoing extraction, concentration, fractionation and/or purification treatments. The combinations thereof are not restricted to any particular one, but examples of a series of treatments are those listed below.

For instance, after extracting olive plants with water and/or a hydrophilic organic solvent, the hydrophilic organic solvent is partially or completely removed from the resulting extract and then the water insolubles separated from the aqueous phase are recovered to thus concentrate the extract. The separated water insolubles can be recovered by means of, for instance, filtration or centrifugation, but the aqueous phase can, if necessary, be subjected to addition of water, stirring or the like to improve the efficiency of the recovery. Moreover, the dried product obtained by removing the water and/or hydrophilic organic solvent from the extract derived from the olive plants can likewise be subjected to the foregoing treatments such as addition of water and stirring and then the resulting water insolubles are recovered through, for instance, filtration to thus concentrate the extract. This concentration method is preferred since it is a treatment in an aqueous system, excellent in safety as compared with the concentration carried out using a solvent other than water and it permits the use of a wide variety of machinery and tools. Moreover, this method is likewise preferred since it is excellent in the concentration-purification efficiency and the resulting product is almost free of any oil component.

Maslinic acid and/or physiologically acceptable salts thereof highly purified can conveniently be obtained by subjecting these concentrates to fractionation-purification treatments according to normal phase and/or reverse phase chromatography and/or recrystallization techniques.

Alternatively, the extract derived from olive plants can be concentrated according to the liquid-liquid partition between water and a hydrophobic organic solvent, which comprises the steps of removing the hydrophilic organic solvent from the extract, adding, if necessary, water to the remaining aqueous solution and then further adding a hydrophobic organic solvent thereto. In addition, the dried product of the extract can likewise be concentrated according to the liquid-liquid partition between water and a hydrophobic organic solvent, which comprises the steps of adding water to the dried product and then adding a hydrophobic organic solvent thereto. Maslinic acid and/or physiologically acceptable salts thereof highly purified can be obtained by subjecting these concentrates to fractionation-purification treatments according to normal phase and/or reverse phase chromatography and/or recrystallization techniques.

It is preferred to prepare an external agent for the skin or a whitening agent using a product isolated from naturally occurring substances since the agent is improved in the percutaneous absorption, any influence of contaminants can be eliminated and the resulting agent is in a colorless to slightly colored and/or odorless to almost odorless condition.

Moreover, the overall content of maslinic acid and physiologically acceptable salts thereof present in the mixture of maslinic acid and physiologically acceptable salts thereof prepared from olive plants and/or the product obtained in or during the olive oil production processes is preferably not less than 95% by mass and more preferably 95 to 99.99% by mass. This content can be determined by, for instance, gas chromatography.

The external agent for the skin and whitening agent according to the present invention comprise maslinic acid and/or physiologically acceptable salts thereof, but may likewise comprise the foregoing extract and concentrate. Moreover, the concentration or the like of maslinic acid and/or physiologically acceptable salts thereof in the extract can be adjusted by controlling the degrees of, for instance, concentration and purification and the resulting product can conveniently be incorporated into the foregoing agents. More specifically, the extract is concentrated when a stronger effect is required, while if a stronger effect is not necessary, the extract may be diluted. In other words, these agents may be prepared such that they contain the effective component in a variety of concentrations depending on the purposes.

Furthermore, the extract can likewise be used in combination with other substances having whitening effects, this allows the detailed design of the whitening effect and the whitening effect of the resulting agent may significantly be improved due to the synergistic effect with other substances having whitening effects. More specifically, the whitening effect of a particular agent can be designed by appropriately controlling the intensity of the whitening effect and efficacy of the extract. The intensity of the whitening effect can be controlled by concentrating the extract when a stronger effect is required or by diluting the extract when a stronger effect is not needed prior to the incorporation thereof into each agent. In other words, the intensity of the whitening effect can appropriately be adjusted depending on purposes. Moreover, the intensity of the whitening effect can likewise be adjusted by appropriately combining the whitening components of the present invention such as maslinic acid with other whitening components. The efficacy of the whitening effect specifically include, for instance, the prevention of sunburn through the shielding of UV light rays, the prevention

of any pigmentation or precipitation of pigments caused after the sunburn and the effect of improving the pigmented skin. The efficacy may likewise be controlled by appropriately combining the whitening components of the present invention such as maslinic acid with other whitening components.

Olive oil comprises maslinic acid and therefore, the oil is preferably used in the external agent for the skin and whitening agent of the present invention as an oil component or medicinal oil to achieve a further preferred whitening effect.

Moreover, the use of seeds containing oil components in the extraction permits the preparation of a water-soluble composition containing such oil components. The oil-containing water-soluble composition extracted from seeds containing oil components can suitably be used in case where the product to be prepared requires the use of oil components and the resulting product may be a composition completely derived from olive and having a whitening effect. As has been described above, olive oil comprises maslinic acid and therefore, it is a preferred embodiment of the present invention to use the composition in, for instance, the external agent of the present invention.

Moreover, an emulsified composition completely derived from olive can be prepared by the addition of an emulsifying agent to the oil-containing water-soluble composition and the emulsified composition may be incorporated into the external agent for the skin and whitening agent of the invention. In particular, the external agent for the skin and whitening agent are frequently used in the form of an emulsion and therefore, the emulsified composition can be incorporated into these agents or may suitably be used as a base to which other substances are to be added.

In addition, the emulsified composition is almost completely derived from olive or derived from naturally occurring substances and therefore, it would give higher feeling of security to the consumers as compared with synthetic products. Moreover, olive in general gives a favorable impression and the composition in turn gives good impression and feeling of security to the users.

Moreover, the present invention makes the most use of the whole olive plant and provides a favorable method for effectively using the same.

Examples of the foregoing emulsifying agents are nonionic ones such as glycerin monostearate, polyoxyethylene (POE) sorbitan fatty acid esters, polyoxyethylene (POE) sorbit fatty acid esters, sorbitan fatty acid esters, POE alkyl ethers, POE polyoxypropylene (POP) block copolymers and POE-hardened castor oil esters and anionic ones such as fatty acid soap and sodium alkylsulfates.

Moreover, the external agent for the skin and whitening agent according to the present invention may also comprise other pharmaceutically effective agents. Specific examples of such pharmaceutical agents are those listed below.

Whitening agents other than those of the present invention include, for instance, vitamin C and derivatives and salts thereof, arbutin, glutathione, placenta extracts, extracts of strawberry geranium, extracts of coix seeds, extracts of scutellaria roots, extracts from marine algae and wheat extracts.

Among these whitening agents, particularly preferred are vitamin C and derivatives and salts thereof, arbutin, glutathione and placenta extracts.

Examples of antioxidants usable herein include enzymes such as superoxide dismutase, catalase and glutathione peroxidase, tocopherol and derivatives thereof; dibutyl hydroxytoluene, butyl hydroxyanisole; carotenoids and derivatives thereof such as β -carotene; tannin and derivatives thereof such as gallic acid and ellagic acid; flavonoids such as flavone, catechin, quercetin and leucoanthocyanidin; quinones such as ubiquinone and vitamin K; thiamines and salts thereof; riboflavins such as riboflavin and riboflavin acetate; pyridoxines such as pyridoxine hydrochloride and pyridoxine dioctanoate; nicotinic acids such as nicotinic acid amide and benzyl nicotinate; bilirubin, mannitol, tryptophane, histidine and nordihydroguaiaretic acid.

Among these antioxidants, particularly preferred are superoxide dismutase, tocopherol and derivatives thereof, quercetin and mannitol.

Examples of anti-inflammatory agents are glycyrrhizic acid, glycyrrhetic acid, allantoin, azulene, mefenamic acid, phenylbutazone, indometacin, ibuprofen, ketoprofen, ε-aminocaproic acid, hydrocortisone, panthenol and derivatives and salts thereof, zinc oxide, diclofenac sodium, aloe extract, extract of beefsteak plant, mugwort extract, camomile extract, comfrey extract, sanguisorba root extract and water-cress extract.

Particularly preferred anti-inflammatory agents are, for instance, glycyrrhizic acid, glycyrrhetic acid, derivatives thereof and salts thereof, among others.

As cell activators, there may be listed, for instance, royal jelly, photo-sensitizers, cholesterol and derivatives thereof fetal calf blood extract, vitamin A and derivatives thereof, citric acid, lactic acid, tartaric acid, malic acid, glycolic acid, succinic acid, serine, glutamic acid, hydroxyproline, theanine, pyrrolidone carboxylic acid, yeast extract, lactobacillus extract, Bifidobacterium bifidum extract and fermented metabolic extract.

Among these cell activators, particularly preferred are vitamin A and derivatives thereof, citric acid, malic acid, lactic acid, serine and pyrrolidone carboxylic acid.

Examples of UV-screening agents include benzoic acid type UV-screening agents such as p-aminobenzoic acid; anthranilic acid type UV-screening agents such as methyl ester of anthranilic acid; salicylic acid type UV-screening

agents such as methyl salicylate; cinnamic acid type UV-screening agents such as methyl p-methoxy-cinnamate; benzophenone type UV-screening agents such as 2-hydroxy-4-methoxy-benzophenone; urocanic acid type UV-screening agents such as ethyl ester of urocanic acid; 4-t-butyl-4'-methoxybenzoyl methane, 2-(2'-hydroxy-5'-methylphenyl) benzotriazole, oxybenzene, titanium oxide, fine particulate titanium oxide and zinc oxide.

Particularly preferred UV-screening agents are, for instance, methyl p-methoxy-cinnamate, titanium oxide, fine particulate titanium oxide and zinc oxide, among others.

Examples of blood circulation promoters are swertia herb extract, cepharanthine, tocopherol and derivatives thereof, nicotinic acid and derivatives thereof, nonanic acid vanillylamide, capsaicine, zingerone, cantharides tincture, ichthammol, caffeine, tannic acid, $\alpha$-borneol, cyclandelate, cinnarizine, tolazoline, acetylcholine, verapamil, $\gamma$-oryzanol, camphor, hinokitiol, enzymes such as lipases and papain, red pepper extract, swertia herb extract, arnica extract, safflower extract and Japanese angelica root extract.

Among these blood circulation promoters, particularly preferred are, for instance, cepharanthine, tocopherol and derivatives thereof and $\gamma$-oryzanol.

Examples of humectants are polyhydric alcohols such as glycerin, diglycerin, propylene glycol, dipropylene glycol, 1,3-butylene glycol and polyethylene glycol; proteins, derivatives thereof, hydrolyzates thereof and salts thereof such as collagen, elastin and keratin; amino acids and derivatives thereof such as glycine, aspartic acid and arginine; sorbitol, xylitol, erythritol, trehalose, inositol, glucose, sucrose and derivatives thereof; dextrin and derivatives thereof; sugars such as bees honey; D-panthenol and derivatives thereof; hyaluronic acid and salts thereof; mucopolysaccharides such as chondroitin sulfuric acid; pyrrolidone carboxylic acid salts, urea, phospholipids, glycolipids, ceramide and sodium lactate.

Examples of particularly preferred humectants are propylene glycol, proteins such as collagen, elastin and keratin, hyaluronic acid and salts thereof, and mucopolysaccharides such as chondroitin sulfuric acid.

The amounts of pharmaceutical agents to be incorporated into the external agent for the skin and whitening agent according to the present invention may vary depending on the kinds of the pharmaceutical agents, but they are preferably used in amounts falling within the following ranges. If they are used in amounts specified below and in combination with maslinic acid, erythrodiol, uvaol, betulinic acid, betulin and/or physiologically acceptable salts thereof or derivatives thereof, the addition of these pharmaceutical agents would allow the resulting agents to show higher whitening effects and skin-beautifying effects without adversely affecting the agents.

More specifically, the content of whitening agents other than those of the present invention in the external agent for the skin and the whitening agent of the present invention preferably ranges from 0.00001 to 10% by mass and more preferably 0.0001 to 5% by mass. When using them in combination with the whitening agent of the present invention, the ratio (by mass) of these whitening agents to that of the invention preferably ranges from 0; 100 to 99.99: 0.01. When the placenta extract and plant extract are used in the liquid state, it is sufficient to use the same in such a manner that the dry solids content thereof falls within the ranges specified above. If the amount thereof falls within the range specified above, the resulting external agent for the skin has more excellent whitening and skin-beautifying effects and also provides good feeling when applied to the skin.

The amount of the antioxidants to be incorporated into the external agent for the skin or the whitening agent of the present invention preferably ranges from 0.00001 to 5% by mass and more preferably 0.0001 to 3% by mass. In case where the plant extract is used in the liquid state without any post-treatment, it is sufficient to use the same in such a manner that the dry solids content thereof falls within the ranges specified above. If the amount thereof falls within the range specified above, the resulting external agent for the skin has a more excellent antioxidant effect, permits the prevention of any occurrence of inflammation, dark skin and skin-aging due to the formation of lipid peroxides within the skin and shows excellent whitening and skin-beautifying effects.

The amount of the anti-inflammatory agent to be incorporated into the external agent for the skin and the whitening agent of the present invention preferably ranges from 0.00001 to 5% by mass and more preferably 0.0001 to 3% by mass. In case where the plant extract is used in the liquid state without any post-treatment, it is sufficient to use the same in such a manner that the dry solids content thereof falls within the ranges specified above. If the amount thereof falls within the range specified above, the resulting external agent for the skin has an excellent anti-inflammatory effect and shows excellent whitening and skin-beautifying effects.

The amount of the cell activator to be incorporated into the external agent for the skin and the whitening agent of the present invention preferably ranges from 0.00001 to 5% by mass and more preferably 0.0001 to 3% by mass. In case where the plant extract is used in the liquid state without any post-treatment, it is sufficient to use the same in such a manner that the dry solids content thereof falls within the ranges specified above. If the amount thereof falls within the range specified above, the resulting external agent for the skin has an excellent rough skin-curing effect and also shows excellent whitening and skin-beautifying effects.

The amount of the UV-screening agent to be incorporated into the external agent for the skin and the whitening agent of the present invention preferably ranges from 0.01 to 20% by mass and more preferably 0.1 to 10% by mass. If the amount thereof falls within the range specified above, the resulting external agent for the skin has an excellent

UV-screening effect and also shows excellent whitening and skin-beautifying effects.

The amount of the blood circulation promoter to be incorporated into the external agent for the skin and the whitening agent of the present invention preferably ranges from 0.001 to 10% by mass and more preferably 0.01 to 20% by mass. If the amount thereof falls within the range specified above, the resulting external agent for the skin has an excellent blood circulation promoting effect and also shows excellent whitening and skin-beautifying effects.

The amount of the humectant to be incorporated into the external agent for the skin and the whitening agent of the present invention preferably ranges from 0.001 to 70% by mass and more preferably 0.1 to 20% by mass. If the amount thereof falls within the range specified above, the resulting external agent for the skin has an excellent moisturizing effect and also shows excellent whitening and skin-beautifying effects.

These whitening agents, antioxidants, anti-inflammatory agents, cell activators, UV-screening agents, blood circulation promoters and humectants may be used alone or in any combination of at least two of them.

The external agent for the skin according to the present invention can be prepared by incorporating the extract into a base in a variety of forms or shapes, which have been known as the usual agents externally applied to the skin, according to the currently used method.

The shape (or dosage form) of the formulated external agent for the skin according to the present invention is not restricted to any particular one and specific examples thereof are cosmetics, pharmaceutical agents for external use and quasi-drugs such as milky lotions, creams, toilet waters, packs, foundations, powders, cosmetics for washing and cleansing, cosmetics for make-up, dispersions and ointments. Among these, preferred are milky lotions, creams, toilet waters, packs, foundations and ointments.

The shape of the formulated whitening agent for the skin according to the present invention is not restricted to any particular one. Specific examples thereof are cosmetics, pharmaceutical agents for external use and quasi-drugs such as milky lotions, creams, toilet waters, packs, foundations, powders, cosmetics for washing and cleansing, cosmetics for make-up, dispersions and ointments when the agent is used as an external agent directly applied to the skin. Among these, preferred are milky lotions, creams, toilet waters, packs, foundations and ointments. Moreover, when it is used as a raw material to be incorporated into an external agent for the skin, the shape thereof is not restricted to any specific one inasmuch as it can ensure a desired content in the external agent for the skin. Specific examples thereof are solid powder, solution, dispersion and emulsion states and either of them may suitably be used without any restriction.

Moreover, the external agent for the skin and whitening agent of the present invention may, if necessary, comprise other commonly used components in such an amount that the use thereof never adversely affects the effects of the present invention and specific examples thereof are water (purified water, hot spring water and deep-sea water), oil component or medicinal oils, surfactants, metallic soap, gelatinizing agents, powders, alcohols, water-soluble polymers, film-forming agents, resins, clathrate compounds, antibacterial agents, perfumes, deodorants, salts, pH-controlling agents, refrigerants, extracts derived from plants, animals and microorganisms, astringents, lipid-leakage blocking agents, chelating agents, keratin-solubilizing agents, enzymes, hormones and vitamins. Specific examples of these components preferably used herein will be listed below. In this respect, the "derivative(s)" listed below includes salts capable of being prepared.

The oil component or medicinal oil is used herein for improving the handling ability of the constituents of the base and for improving the feeling upon use, may be any one inasmuch as it is used in the usual cosmetics and may be selected irrespective of, for instance, the origin and the quality thereof. Therefore, it may be a natural oil or a synthetic oil or may be in the form of a solid, a semisolid or a liquid. Examples thereof usable herein are hydrocarbons, waxes, fatty acids, higher alcohols, ester oils, silicone oils and fluorine atom-containing oils.

Specific examples of such oil components or medicinal oils are hydrocarbons such as liquid paraffin, squalane and vaseline; oils and fats derived from plants and animals such as olive oil, castor oil, jojoba oil, mink oil, macademia nuts oil, apricot oil, persic oil, safflower oil, sunflower oil, avocado oil, meduhome oil, camellia oil, almond oil, perilla oil, sesame oil, borage oil, cacao butter, shea butter, lanolin and reduced lanolin; and waxes such as yellow bees wax, carnauba wax, candelilla wax and spermaceti.

The amount of the oil component or medicinal oil to be incorporated into the external agent for the skin and the whitening agent of the present invention preferably ranges from 0.01 to 90% by mass and more preferably 0.1 to 50% by mass.

The surfactant is used for emulsification or solubilization of, for instance, oil component or medicinal oil and examples thereof usable herein are anionic, cationic, nonionic and amphoteric surfactants.

The metallic soap may be, for instance, metal salts other than alkali metal salts of fatty acids and specific examples thereof are aluminum stearate, magnesium stearate and zinc laurate.

The gelatinizing agent is used for the stabilization of the resulting system, for improving the handling ability of the constituents of the base and for improving the feeling upon use and examples thereof include amino acids derivatives such as N-lauroyl-L-giutamic acid, dextrin fatty acid esters such as dextrin palmitic acid esters, sucrose esters of fatty acids and clay minerals modified with organic substances.

The powder is mainly used for blocking a color or the skin in the cosmetics for make-up and for a wide variety of other purposes, for instance, for improving the feeling upon use and may be any powder inasmuch as it is commonly used in the usual cosmetics, irrespective of the shape (such as spherical, needle-like, plate-like shapes), particle size (fume, fine particles, pigment size) and/or particle structure (porous, nonporous). Specific examples thereof include inorganic powders such as barium sulfate, calcium carbonate, talc, mica, synthetic mica, mica, kaolin, sericite, silicic acid, silicic acid anhydride, aluminum magnesium silicate, ceramics powders and boron nitride; organic powders such as polyester powders, polyethylene powders, polystyrene powders, nylon powders and lauroyl lysine; colored pigments, for instance, inorganic pigments such as iron oxide, carbon black, chromium oxide, iron blue and ultramarine; lake pigments of tar-containing dye and lake pigment of natural dye; pearl pigments such as titanium oxide-coated mica, titanium oxide-coated mica, bismuth oxychloride, titanium oxide-coated bismuth oxychloride, titanium oxide-coated talc, fish scale flakes and titanium oxide-coated pigmented mica; other tar dyes; and naturally occurring dyes such as cochineal. These powders may be combined to form composites or may be surface-treated with oil component or medicinal oils, silicone or fluorine atom-containing compounds.

Examples of alcohols are lower alcohols such as ethanol and isopropanol; glycerin, diglycerin, ethylene glycol, diethylene glycol, triethylene glycol, propylene glycol, dipropylene glycol, 1,3-butylene glycol and polyethylene glycol.

The water-soluble polymer is used for improving the stability of the resulting system, the handling ability and the feeling upon use thereof or for imparting moisturizing effect to the system. Specific examples of water-soluble polymers are polymers derived from plants such as carrageenan, pectin, agar and locust bean gum; polymers derived from microorganisms such as xanthan gum; polymers derived from animals such as casein and gelatin; starch type polymers such as starch; cellulose type polymers such as methyl cellulose, ethyl cellulose, carboxymethyl cellulose, hydroxypropyl cellulose, nitrocellulose and crystalline cellulose; alginic acid type polymers such as sodium alginate; vinylic polymers such as carboxyvinyl polymer; polyoxyethylene type polymers; polyoxyethylene polyoxypropylene copolymer type polymers; acrylic polymers such as sodium polyacrylate; and inorganic water-soluble polymers such as bentonite and hectorite. The water-soluble polymer also includes film-forming agents such as polyvinyl alcohol and polyvinyl pyrrolidone. Among these, preferred are carboxyvinyl polymer, polyvinyl alcohol and carboxymethyl cellulose.

Examples of extracts derived from animals or microorganisms include protein free of serum, spleen, egg components derived from, for instance, fowls, chorion extract, cockscomb extract, shell extract, shellfish meat extract, royal jelly, silk protein and decomposition products thereof or derivatives thereof, hemoglobin and decomposition products thereof, lactoferrin or decomposition products thereof, mollusks such as cuttlefish ink, fish meat, extracts derived from animals such as mammals, fowls, shellfishes, insects, fishes, mollusks and crustaceans and extracts derived from microorganisms such as extract of Fomes japonicus. Various effects such as a moisturizing effect, a cell-activating effect, a whitening effect, an anti-inflammatory effect, an anti-aging effect for the skin, an active oxygen-elimination effect and a blood circulation-promoting effect can be imparted to the extract and/or the external agent for the skin and the whitening agent by the addition of an extract derived from an animal or a microorganism thereto.

The extracts derived from plants are not limited to the sites to be extracted and methods for the extraction and may be those extracted from, for instance, roots, stems, trunks, barks, plumules, leaves, flowers, fruits and seeds. In this respect, the extraction can be conducted, for instance, by subjecting these raw materials to appropriate treatments such as drying, finely cutting, pressing or fermentation and then extracting these treated raw materials with a variety of appropriate solvents at a low temperature, room temperature or under a gently heated conditions. Examples of extraction solvents are water; lower primary alcohols such as methyl alcohol and ethyl alcohol; liquid polyhydric alcohols such as glycerin, propylene glycol and 1,3-butylene glycol, which may be used alone or in any combination. It is also possible to conduct the extraction using a lipophilic solvent such as hexane, acetone, ethyl acetate and ether or the extract may likewise be one obtained by extracting with an oily component such as squalane. The resulting extract may further be subjected to absorption, discoloration and/or purification using filtration and/or an ion-exchange resin to thus convert the extract into, for instance, a solution, a paste, a gel or a powder. If necessary, the extract thus treated may further be subjected to a purification treatment such as deodorization and/or discoloration inasmuch as the treatments never adversely affect the effects of the extract. Examples of plants as sources for the extract are asparagus, rose fruits, raspberry, sophora roots, Spatholobus suberectus Dunn, acanthopanacis cortex, coffee, rice plant, asiasarum roots, crataegus, white lily, herbaceous peony, tea plant, Japanese beech, hops, Japanese dwarf quince, Saxifraga stronifera Meerburg, marshmallow, Angelica keiskei, Koidz, Artemisia capillaries herba, nettle, phellodendron bark, Hypericum erectum, lonicerae flos, Salvia officinalis, lithospermum roots, white birch, Sapindus mukurossi Gaertn., Chinese milk vetch, barley, jujube, rosemary, coptis rhizome, grapefruit, gentian, Saponaria officinalis L., Japanese iris, rehmannia roots, cnidium rhizome, tree mallow, witch-hazel (leaves), coltsfoots, Japanese (and Chinese) plum, Tilia miqueliana, horse-chestnut and Cydonia oblonga Mill. Various effects such as a moisturizing effect, a cell-activating effect, a whitening effect, an anti-inflammatory effect, an anti-aging effect for the skin, an active oxygen-elimination effect and a blood circulation-promoting effect can be imparted to the extract and/or the external agent for the skin and the whitening agent by the addition of an extract derived from a plant thereto.

Examples of antibacterial agents are benzoic acid, sodium benzoate, p-oxybenzoic acid esters, p-chloro-m-cresol, benzalkonium chloride, phenoxy ethanol and isopropyl methyl phenol.

In addition, the external agent for the skin and whitening agent of the present invention may likewise comprise, if necessary, other currently used additives such as a preservative, a dye, a thickening agent, a perfume and/or a beautifying component.

Moreover, the external agent for the skin and whitening agent of the present invention may be formed into pharmaceutical preparations together with, for instance, saccharides such as lactose and glucose; excipients such as dextrin, cellulose, silicates (salts) and calcium carbonate; binders such as cellulose, starch, gelatin and saccharides; and disintegrating agents such as agar powder, gelatin powder, sodium alginate, cellulose derivatives and calcium carbonate. Further, these preparations may be in any form such as liquids, solids, semisolids, emulsions and mousse.

The external agent for the skin of the present invention can be prepared according to the usual method and may have any form or dosage form such as a milk lotion, a cream, a toilet water, a beauty lotion, a cleansing, a pack, a cosmetic for washing or cleansing and a foundation, as well as other dispersions, granules and ointments.

In addition, maslinic acid and/or physiologically acceptable salts thereof are those suitably prepared from olive plants, as has been discussed above, and therefore, it may sometimes comprise other components originated from the olive plants. Examples of such other components originated from the olive plants are triterpenes such as oleanolic acid and ursolic acid, sterols such as β-sitosterol and campesterol, and polyphenols such as Oleuropein, Verbascosid, Ligstrosid, tyrosol, hydroxytyrosol and rutin.

The foregoing components may, if necessary, be incorporated into the external agent for the skin or the whitening agent of the present invention to thus impart the effects of these components to the latter.

In particular, the external agent for the skin or the whitening agent of the present invention, which comprise maslinic acid, physiologically acceptable salts thereof and/or derivatives thereof can be prepared by, in particular, a method comprising the steps of extracting olive plants and/or the products obtained in or during the olive oil production processes with water and/or an organic solvent, subjecting the resulting extract to a concentration treatment and/or a fractionation-purification treatment to thus give maslinic acid, physiologically acceptable salts thereof and/or derivatives thereof, optionally derivatizing the product thus obtained, and then admixing the product with at least one oil component or medicinal oil and at least one nonionic surfactant.

Moreover, the toilet water of the present invention, which comprise maslinic acid, physiologically acceptable salts thereof and/or derivatives thereof can be prepared by a method comprising the steps of extracting olive plants and/or the products obtained in or during the olive oil production processes with water and/or an organic solvent, subjecting the resulting extract to a concentration treatment and/or a fractionation- purification treatment to thus give maslinic acid, physiologically acceptable salts thereof and/or derivatives thereof and then admixing the product with at least one alcohol.

Accordingly, the present invention relates to a whitening agent comprising at least one member selected from the group consisting of maslinic acid, erythrodiol, uvaol, betulinic acid, betulin and physiologically acceptable salts thereof, which are prepared from natural plants containing each corresponding penta-cyclic triterpene according to the similar method or at least one member selected from the group consisting of derivatives of the foregoing compounds as an effective component. The present invention also relates to an external agent for the skin comprising the foregoing whitening agent.

The external agent for the skin or the like according to the present invention comprising at least one member selected from the group consisting of maslinic acid, erythrodiol, uvaol, betulinic acid, betulin and/or physiologically acceptable salts or derivatives thereof has a significantly excellent whitening effect and is highly safe for the skin. Regarding, in particular, maslinic acid, preferably maslinic acid and/or physiologically acceptable salts thereof are natural components derived from olive plants and highly safe for the human bodies. In addition, the natural source thereof is olive plants, which have widely been used for food and therefore, the stable supply thereof can be ensured. In addition, it would be recognized that the cosmetics used almost everyday preferably mainly comprise natural components highly safe for the skin and that the users can use the same without any anxiety. Moreover, the fact that these components are derived from olive plants having favorable impression would provide mentally refreshed feeling and feeling of security to the users.

## Examples

Then the present invention will be described with reference to the following Examples, but the present invention is not restricted to these specific Examples at all.

Regarding the penta-cyclic triterpenes used in Examples, erythrodiol (available from Funakoshi Company), uvaol (available from Funakoshi Company), betulinic acid (available from Funakoshi Company) and betulin (available from Funakoshi Company) were purchased as reagents. In this connection, those of HPLC grade were used as such without any post-treatment, while the remaining reagents were dissolved in ethanol heated to the boiling point thereof till the resulting solution was saturated, followed by recrystallization through cooling, filtration and drying up the recrystallized product before practically using the same. As will be described below while taking an example, maslinic acid used herein was extracted from olive plants and then purified. The purity of the product was confirmed to be 95%.

[Preparation Example 1]

Dried fruits (including seeds) (500 g) of native olive (Olea europaea L.) were crushed and 3 L of hexane was added to the crushed, dried fruits to conduct the extraction thereof for 3 hours. The foregoing operations were repeated 4 times to obtain defatted fruits, the seeds were removed from the fruits, the fruits thus treated were pulverized and the fruits were again extracted with 5 volumes of hexane for 3 hours to give 229 g of defatted lees from which the oil components were completely removed. To the defatted lees, there was added 10 volumes of water-containing ethanol having an ethanol content of 60% by mass, followed by the extraction at room temperature for 3 hours with vigorous stirring. The whole of the extraction system was filtered and the resulting filtrate was concentrated to dryness to give 112.7 g of an extract.

To 100 g of this extract, there was added 2 L of water, followed by vigorous stirring at room temperature for one hour. The whole of the mixture was centrifuged, the resulting supernatant was removed through decantation and the resulting precipitates were dried to give 10.0 g of a concentrate.

Then the concentrate was fractionated by silica gel column chromatography using a column packed with about 40 volumes (400 g) of silica gel. After first eluting various kinds of unnecessary components using, as an elute, a 3: 1 hexane-ethyl acetate mixed solvent in a volume of 10 times (4000 mL) that of the packed gel, various kinds of unnecessary components were eluted using an elute or 2.5 volumes (1000 mL) of a 1: 1 hexane-ethyl acetate mixed solvent. Subsequently, the intended maslinic acid was eluted with an elute or a 1: 1 hexane-ethyl acetate mixed solvent in a volume of 10 times (4000 mL) that of the packed gel to give a crude maslinic acid-containing fraction. The hexane and ethyl acetate were removed from the resulting fraction, followed by drying in a vacuum to give 1.96 g of a crude maslinic acid-containing fraction.

Further, the crude maslinic acid-containing fraction was purified by ODS column chromatography using a column packed with about 30 volume (60 g) of octadecyl-treated silica gel. First of all, various kinds of unnecessary components were eluted with an elute or an 8: 2 methanol-water mixed solvent in a volume of 10 times (600 mL) that of the packed gel. Then the intended maslinic acid was eluted with an elute or an 8: 2 methanol-water mixed solvent in a volume of 30 times (1800 mL) that of the packed gel to thus give a purified maslinic acid-containing fraction. After removing the methanol from the resulting fraction, followed by drying in a vacuum to give 1.51 g of purified maslinic acid 1.

The purified maslinic acid 1 was analyzed by, for instance, NMR and MS spectroscopy and as a result, it was confirmed that a part of the purified maslinic acid 1 was in sodium and potassium salts and the remaining majority thereof was in the free acid state. Moreover, the purity thereof was determined by GC and it was thus confirmed that the purity thereof for maslinic acid was not less than 95%.

[Preparation Example 2]

Olive plants (Olea europaea L.) of Italy growth were subjected to oil expression to give 1 kg of an oil expression residue, 10 volumes of a water-containing ethanol having an ethanol content of 65% by mass was added to the residue and the extraction thereof was conducted at room temperature with vigorous stirring over 3 hours. The whole of the extraction system was filtered and the filtrate was concentrated to dryness to give 20.2 g of an extraction product.

To this extract, there were added 1 L of n-butanol and 1 L of water and the resulting mixture was stirred for 10 minutes to thus separate the mixture into an n-butanol phase and a water phase. The n-butanol was removed from the n-butanol phase, followed by drying in a vacuum to give 13.3 g of a concentrate.

Then the concentrate was fractionated by silica gel column chromatography using a column packed with about 40 volumes (500 g) of silica gel. More specifically, after first eluting various kinds of unnecessary components using, as an elute, a 3: 1 hexane-ethyl acetate mixed solvent in a volume of 10 times (5000 mL) that of the packed gel, various kinds of unnecessary components were eluted using an elute or 2.5 volumes (1250 mL) of a 1: 1 hexane-ethyl acetate mixed solvent. Subsequently, the intended maslinic acid was eluted with an elute or a 1: 1 hexane-ethyl acetate mixed solvent in a volume of 10 times (5000 mL) that of the packed gel to give a crude maslinic acid-containing fraction. The hexane and ethyl acetate were removed from the resulting fraction, followed by drying in a vacuum to give 2.66 g of a crude maslinic acid-containing fraction.

Further, the crude maslinic acid-containing fraction was purified by ODS column chromatography using a column packed with about 30 volume (80 g) of octadecyl-modified silica gel. First of all, various kinds of unnecessary components were eluted with an elute or an 8: 2 methanol-water mixed solvent in a volume of 10 times (800 mL) that of the packed gel. Then the intended maslinic acid was eluted with an elute or an 8: 2 methanol-water mixed solvent in a volume of 30 times (2400 mL) that of the packed gel to thus give a purified maslinic acid-containing fraction. After

removing the methanol from the resulting fraction, followed by drying in a vacuum to give 2.06 g of purified maslinic acid 2.

The purified maslinic acid 2 was analyzed by, for instance, NMR and MS spectroscopy and as a result, it was confirmed that a part of the purified maslinic acid 2 was in the free acid state and the remaining majority thereof was in the form of salts with, for instance, sodium and potassium. Moreover, the purified maslinic acid 2 was inspected for the purity thereof by GC and it was confirmed that the purity thereof for maslinic acid was not less than 97%.

Derivatives of penta-cyclic triterpenes were prepared as follows:

[Synthesis 1]: Ethyl Ester of Maslinic Acid

Maslinic acid (4.5 g) and triethylamine (1.0 g) were dissolved in 50 mL of chloroform and then a solution of thionyl chloride (1.1 g) in 10 mL of chloroform was dropwise added to the resulting solution with ice cooling and stirring for one hour. Then 3.2 g of ethanol was added to the mixture and a solution of triethylamine (1.0 g) in 10 mL of chloroform was dropwise added to the mixture with ice cooling and stirring for 3 hours. After the completion of the reaction, the fraction soluble in chloroform was extracted, followed by distillation of the chloroform to give a crude reaction product and purification thereof with silica gel column chromatography to give 3.5 g of ethyl ester of maslinic acid.

[Synthesis 2]: 2,3-O-Di-Acetyl-Maslinic Acid

Maslinic acid (2.0 g) was dissolved in 100 mL of pyridine, 50 mL of acetic acid anhydride was added to the solution and the mixture was stirred overnight. After distilling off the pyridine and acetic acid anhydride, the resulting residue was dissolved in ether, the resulting ether phase was washed once with a 1N hydrochloric acid aqueous solution, once with a saturated sodium hydrogen carbonate solution and 3 times with pure water, followed by addition of magnesium sulfate and allowing to stand overnight. The magnesium sulfate was removed through filtration, the ether was distilled off to give a crude product and the latter was purified by silica gel column chromatography to give 2.2 g of 2,3-O-di-acetyl-maslinic acid.

[Synthesis 3]: Triethylsilyl Ester of 2,3-O-Di-Triethylsilyl-Maslinic Acid

Maslinic acid (1.0 g) was dissolved in 200 mL of anhydrous dimethylformamide, 144.0 mg of imidazole and 350 μL of triethylsilyl chloride were added to the resulting solution at 0°C, the mixture was sealed with a cap and stirred for 2 hours. The dimethylformamide was distilled off, the resulting residue was dissolved in ether, the ether phase was washed once with a 1N hydrochloric acid aqueous solution, once with a saturated sodium hydrogen carbonate solution and 3 times with pure water, followed by addition of magnesium sulfate and allowing to stand overnight. The magnesium sulfate was removed through filtration, the ether was distilled off to give a crude product and the latter was purified by silica gel column chromatography to give 1.5 g of triethylsilyl ester of 2,3-O-di- triethylsilyl-maslinic acid.

[Synthesis 4]: Ethyl Ester of 2,3-O-Di-Stearoyl-Maslinic Acid

The ethyl ester of maslinic acid (1.0 g) prepared in Synthesis 1 was dissolved in 50 mL of anhydrous toluene, 5.0 g of triethylamine was added to the resulting solution, 6.0 g of stearic acid chloride was gradually added to the mixture with ice cooling and stirring for one hour and the resulting mixture was stirred over 9 hours while the temperature thereof was gradually reduced down to room temperature. To the mixture, there was added a proper amount of a 1N hydrochloric acid aqueous solution, the resulting mixture was extracted with ether, the ether phase was washed once with a saturated sodium hydrogen carbonate solution and 3 times with pure water, followed by addition of magnesium sulfate and allowing to stand overnight. The magnesium sulfate was removed through filtration, the ether was distilled off to give a crude product and the latter was purified by silica gel column chromatography to give 1.2 g of ethyl ester of 2,3-O-di-stearoyl-maslinic acid.

[Synthesis 5]: 3,28-O-Di-Acetyl-Erythrodiol

Erythrodiol (5.0 g) was dissolved in 250 mL of pyridine, 100 mL of acetic anhydride was added to the resulting solution and the mixture was allowed to stand overnight. After the pyridine and acetic anhydride were distilled off, the resulting residue was dissolved in ether, the resulting ether phase was washed once with a 1N hydrochloric acid aqueous solution, once with a saturated sodium hydrogen carbonate solution and 3 times with pure water, magnesium sulfate was added to the ether phase and the latter was allowed to stand overnight. The magnesium sulfate was removed through filtration, the ether was distilled off to give a crude product and the latter was purified by silica gel column chromatography to give 5.4 g of 3,28-O-di-acetyl- erythrodiol.

[Synthesis 6]: 3,28-O-Di-Acetyl-Uvaol

Uvaol (5.0 g) was dissolved in 250 mL of pyridine, 100 mL of acetic anhydride was added to the resulting solution and the mixture was allowed to stand overnight. After the pyridine and acetic anhydride were distilled off, the resulting residue was dissolved in ether, the resulting ether phase was washed once with a 1N hydrochloric acid aqueous solution, once with a saturated sodium hydrogen carbonate solution and 3 times with pure water, magnesium sulfate was added to the ether phase and the latter was allowed to stand overnight. The magnesium sulfate was removed through filtration, the ether was distilled off to give a crude product and the latter was purified by silica gel column chromatography to give 5.4 g of 3,28-O-di-acetyl-uvaol.

[Synthesis 7]: Ethyl Ester of Betulinic Acid

Betulinic acid (5.0 g) and triethylamine (1.1 g) were dissolved in 50 mL of chloroform and a solution of thionyl chloride (1.2 g) in chloroform (10 mL) was dropwise added to the resulting solution with ice cooling and stirring for one hour. Subsequently, 3.5 g of ethanol was added to the mixture and a solution of triethylamine (1.1 g) in chloroform (10 mL) was dropwise added to the mixture with ice cooling and stirring over 3 hours. After the completion of the reaction, the moiety soluble in chloroform was extracted, the chloroform was distilled off to give a crude reaction product and the latter was purified by silica gel column chromatography to give 3.8 g of ethyl ester of betulinic acid.

[Synthesis 8]: 3,28-O-Di-Acetyl-Betulin

Betulin (5.0 g) was dissolved in 250 mL of pyridine, 100 mL of acetic anhydride was added to the resulting solution and the mixture was allowed to stand overnight. After the pyridine and acetic anhydride were distilled off, the resulting residue was dissolved in ether, the resulting ether phase was washed once with a 1N hydrochloric acid aqueous solution, once with a saturated sodium hydrogen carbonate solution and 3 times with pure water, magnesium sulfate was added to the ether phase and the latter was allowed to stand overnight. The magnesium sulfate was removed through filtration, the ether was distilled off to give a crude reaction product and the latter was purified by silica gel column chromatography to give 5.4 g of 3,28-O-di-acetyl-betulin.

Then we will explain a method for determining the melanogenesis-inhibitory function in a cell culture system using B-16 melanoma cells and a method for determining the cell survival rate.

[Test Example]: Test for Evaluating Melanogenesis-Inhibitory Function in Cell Culture Systems Using B-16 Melanoma Cells

A culture medium was dispensed to wells of a 6-well plate (2 mL/well each), followed by inoculation of a desired amount of B-16 melanoma cells on each well, allowing the wells to stand at 37°C and 5% $CO_2$ to thus cultivate the cells. On the day subsequent thereto, a sample solution prepared was added to the wells with stirring and the cultivation was continued. The culture medium was replaced with fresh one on the $5^{th}$ day from the initiation of the cultivation and the sample solution was again added to the wells. On the next day, the culture medium was removed to recover the cells, followed by washing them with PBS (phosphate buffered physiological saline) and then evaluation of the cell-whitening degree. In this respect, the melanogenesis-inhibitory function was evaluated by comparing the cell-whitening degree thus obtained with that observed when the same procedures used above were repeated except that vitamin C-magnesium phosphate as a known whitening agent (positive control) was substituted for the sample solution used above and that observed when the same procedures used above were repeated except that any sample was not added (control), according to the following evaluation criteria.

The evaluation criteria for the cell-whitening degree are as follows:

| (Evaluation Criteria) | |
|---|---|
| Evaluation | Details |
| ++ | The whitening degree is higher than that observed when adding 450 μg/mL of vitamin C-magnesium phosphate. |
| + | The whitening degree is almost identical to that observed when adding 450 μg/mL of vitamin C-magnesium phosphate. |
| ± | The whitening degree is not higher than that observed when adding 450 μg/mL of vitamin C-magnesium phosphate, but higher than that of the control. |

(continued)

| (Evaluation Criteria) | |
|---|---|
| Evaluation | Details |
| - | The whitening degree is almost identical to that observed for the control. |

[Cell-Survival Rate]

The cell-survival rate at each added sample concentration can be determined according to the following formula:

$$\text{Cell-Survival Rate (\%)} = (A/B) \times 100$$

Wherein A represents the viable cell count observed for the system to which a sample is added and B represents the number of cells observed for the control system.

The purified maslinic acid 1, purified maslinic acid 2, erythrodiol, uvaol, betulinic acid, betulin and compounds prepared in the foregoing Synthetic Examples were evaluated for the degree of whiteness (whitening degree) and cell-survival rate at various concentrations specified in the following Tables 1 and 2 according to the foregoing methods. The results thus obtained are summarized in Tables 1 and 2.

[Results of Whitening Degree-Evaluation]

**Table 1**: Present Invention

| Test Sample | Concentration (ppm) | | | | | | | |
|---|---|---|---|---|---|---|---|---|
| | 2 | 4 | 6 | 8 | 10 | 15 | 25 | 50 |
| Purified maslinic acid 1 | ± | + | ++ | ++ | ++ | | | |
| Purified maslinic acid 2 | ± | + | ++ | ++ | ++ | | | |
| Erythrodiol | ± | ± | ± | ± | ± | ± | + | + |
| Uvaol | - | - | - | ± | + | + | + | + |
| Betulinic acid | - | ± | ± | ± | + | + | + | + |
| Betulin | - | - | ± | ± | ± | + | + | + |
| Compound of Synthesis 1 | ± | + | ++ | ++ | ++ | | | |
| Compound of Synthesis 2 | ± | ± | + | ++ | ++ | ++ | | |
| Compound of Synthesis 3 | - | ± | ± | + | + | + | + | + |
| Compound of Synthesis 4 | ± | ± | + | ++ | ++ | ++ | | |
| Compound of Synthesis 5 | ± | ± | ± | ± | ± | ± | + | + |
| Compound of Synthesis 6 | - | - | - | ± | + | + | + | + |
| Compound of Synthesis 7 | - | ± | ± | ± | + | + | + | + |
| Compound of Synthesis 8 | - | - | ± | ± | ± | + | + | + |

**Table 1 (continued)**: Comparative Example

| Test Sample | Concentration (ppm) | | | | | | | |
|---|---|---|---|---|---|---|---|---|
| | 10 | 15 | 25 | 50 | 100 | 200 | 300 | 400 |
| Arbutin | - | - | - | ± | ± | + | ++ | ++ |
| Kojic Acid | - | - | - | ± | ± | + | ++ | ++ |

(*): The concentration of vitamin C-magnesium phosphate as the positive control was set at 450 ppm.

[Cell-Survival Rate]

**Table 2**: Present Invention

| Test Sample | Concentration (ppm) | | | | | | | | | |
|---|---|---|---|---|---|---|---|---|---|---|
| | 2 | 4 | 6 | 8 | 10 | 15 | 25 | 50 | 100 | 200 |
| Purified maslinic acid 1 | 100 | 100 | 100 | 79 | 57 | | | | | |
| Purified maslinic acid 2 | 100 | 100 | 100 | 88 | 69 | | | | | |
| Erythrodiol | 100 | 100 | 100 | 100 | 100 | 100 | 100 | 97 | 85 | 72 |
| Uvaol | 100 | 100 | 100 | 100 | 100 | 100 | 90 | 82 | 71 | 61 |
| Betulinic acid | 100 | 100 | 100 | 100 | 99 | 83 | 76 | 69 | 60 | 51 |
| Betulin | 100 | 100 | 100 | 100 | 100 | 100 | 100 | 100 | | |
| Compound of Synthesis 1 | 100 | 100 | 100 | 77 | 54 | | | | | |
| Compound of Synthesis 2 | 100 | 100 | 100 | 99 | 80 | 59 | | | | |
| Compound of Synthesis 3 | 100 | 100 | 100 | 100 | 100 | 100 | 100 | 94 | | |
| Compound of Synthesis 4 | 100 | 100 | 100 | 100 | 82 | 60 | | | | |
| Compound of Synthesis 5 | 100 | 100 | 100 | 100 | 100 | 100 | 100 | 100 | 95 | 81 |
| Compound of Synthesis 6 | 100 | 100 | 100 | 100 | 100 | 100 | 100 | 91 | 80 | 69 |
| Compound of Synthesis 7 | 100 | 100 | 100 | 100 | 98 | 81 | 72 | 60 | 52 | |
| Compound of Synthesis 8 | 100 | 100 | 100 | 100 | 100 | 100 | 100 | 100 | | |

**Table 2 (continued)**: Comparative Example

| Test Sample | Concentration (ppm) | | | | | | | |
|---|---|---|---|---|---|---|---|---|
| | 10 | 15 | 25 | 50 | 100 | 200 | 300 | 450 |
| Arbutin | 100 | 100 | 100 | 86 | 69 | 56 | 41 | 16 |
| Kojic Acid | 100 | 100 | 100 | 100 | 100 | 93 | 80 | 66 |

Regarding the results listed in Table 1, if comparing the whitening effects of the purified maslinic acids 1, 2 with that observed for vitamin C-magnesium phosphate (conc.: 450 ppm) as the positive control, the maslinic acids 1, 2 show the whitening effect almost identical to that of the positive control at an added concentration of 4 ppm and therefore, it was confirmed that the purified maslinic acids 1, 2 had a whitening effect of about 110 times that observed for vitamin C-magnesium phosphate. Moreover, the whitening effects of the compounds of Synthesis 1 to 4 as derivatives of maslinic acid were almost identical to that of the positive control at an added concentration ranging from 4 to 8 ppm and this indicates that they have a whitening effect of 56 to 110 times that observed for vitamin C-magnesium phosphate.

Similarly, if comparing the results observed for erythrodiol, uvaol, betulinic acid and betulin with that observed for vitamin C-magnesium phosphate (conc.: 450 ppm) as the positive control, the whitening effects of these compounds

were almost identical to that of the positive control at an added concentration ranging from 10 to 25 ppm and this indicates that these compounds have a whitening effect of about 18 to 45 times that observed for vitamin C-magnesium phosphate. Moreover, the whitening effects of the compounds of Synthesis 5 to 8 as derivatives of erythrodiol, uvaol, betulinic acid and betulin were almost identical to that of the positive control at an added concentration ranging from 10 to 25 ppm and this indicates that they have a whitening effect of 18 to 45 times that observed for vitamin C-magnesium phosphate.

Then, regarding the data listed in Table 2, if comparing the rates of cell proliferation with one another, the cell-survival rate of vitamin C-magnesium phosphate having quite low cytotoxicity is 100% at an added concentration of 450 ppm, while the cell-survival rates of the purified maslinic acids 1, 2, which have a whitening degree almost identical to that of the positive control, are 100% at an added concentration of 4 ppm. It is thus concluded that if the foregoing purified maslinic acids 1, 2 are used as whitening agents, they have safety almost identical to that observed for the existing whitening agent or vitamin C-magnesium phosphate. Moreover, all of the compounds of Synthesis 1 to 4 as derivatives of maslinic acid have a cell-survival rate of 100% at an added concentration ranging from 4 to 8 ppm in which they show a whitening degree almost identical to that observed for vitamin C-magnesium phosphate at an added concentration of 450 ppm and this indicates that if they are used as whitening agents, these compounds of Synthesis 1 to 4 have safety almost identical to that observed for the existing whitening agent or vitamin C-magnesium phosphate, which has a quite low cytotoxicity.

Similarly, erythrodiol, uvaol, betulinic acid and betulin show their whitening degree almost identical to that observed for vitamin C-magnesium phosphate (added concentration: 450 ppm) at an added concentration ranging from 10 to 25 ppm and the cell-survival rate thereof at that added concentration ranges from 99 to 100%. This indicates that if erythrodiol, uvaol, betulinic acid and betulin are used as whitening agents, they have safety almost identical to that observed for the existing whitening agent or vitamin C-magnesium phosphate. In addition, the cell-survival rates of the compounds of Synthesis 5 to 8 as derivatives of erythrodiol, uvaol, betulinic acid and betulin range from 98 to 100% at an added concentration at which the whitening degree thereof is almost identical to that observed for vitamin C-magnesium phosphate at an added concentration of 450 ppm and this likewise indicates that if these derivatives prepared in Synthesis 5 to 8 are used as whitening agents, they have safety almost identical to that observed for the existing whitening agent or vitamin C-magnesium phosphate.

As will be seen from the data listed in Tables 1 and 2, all of the purified maslinic acids 1, 2, erythrodiol, uvaol, betulinic acid, betulin and the compounds in Synthetic Examples according to the present invention are superior to arbutin and kojic acid, which have widely been used as whitening agents and in particular, the whitening effects of the purified maslinic acids 1, 2 are considerably excellent. Moreover, the cell-survival rate of arbutin is 56% at an added concentration of 200 ppm and the purified maslinic acids 1, 2, erythrodiol, uvaol, betulinic acid, betulin and the compounds in Synthetic Examples according to the present invention have a cell-survival rate ranging from 98 to 100%, at an added concentration required for ensuring a whitening degree almost identical to that of arbutin. This clearly indicates that the purified maslinic acids 1, 2, erythrodiol, uvaol, betulinic acid, betulin and the compounds in Synthetic Examples according to the present invention have safety for the skin higher than that observed for the existing whitening agent or arbutin. In particular, the purified maslinic acids 1, 2 have a cell-survival rate of 100% at an added concentration of 4 ppm and this clearly indicates that these compounds are highly safe as compared with the existing whitening agent or arbutin. The similar results are likewise observed when comparing them with those of kojic acid.

As has been discussed above in detail, it is recognized that maslinic acid, erythrodiol, uvaol, betulinic acid, betulin and/or physiologically acceptable salts or derivatives thereof have high whitening effects on the order of about 18 to 110 times that observed for vitamin C-magnesium phosphate widely used as an existing whitening agent and in particular, maslinic acid and/or physiologically acceptable salts or derivatives thereof have an unexpectedly higher whitening effect on the order of 110 times. Accordingly, it has been demonstrated that the present invention can provide an external agent for the skin and a whitening agent, which possess such quite excellent whitening effects that have never been experienced.

[Prescription 1]: Cream

Using the purified maslinic acid 1, purified maslinic acid 2, erythrodiol, uvaol, betulinic acid, betulin and compounds prepared in Synthetic Examples, creams each having the composition specified in the following Table 3 were prepared according to the method detailed below and the resulting creams were inspected for the whitening effect. The results thus obtained are likewise listed in Table 3. Moreover, comparative samples were likewise prepared using vitamin C-magnesium phosphate and kojic acid and without using any test sample.

Table 3

| Component (%) | | Product of the present invention | | | | | | | |
|---|---|---|---|---|---|---|---|---|---|
| | | 1 | 2 | 3 | 4 | 5 | 6 | 7 | 8 |
| (1) Bees Wax | | 6.0 | 6.0 | 6.0 | 6.0 | 6.0 | 6.0 | 6.0 | 6.0 |
| (2) Cetanol | | 5.0 | 5.0 | 5.0 | 5.0 | 5.0 | 5.0 | 5.0 | 5.0 |
| (3) Reduced lanolin | | 5.0 | 5.0 | 5.0 | 5.0 | 5.0 | 5.0 | 5.0 | 5.0 |
| (4) Squalane | | 30.0 | 30.0 | 30.0 | 30.0 | 30.0 | 30.0 | 30.0 | 30.0 |
| (5) Glycerin mono | | 4.0 | 4.0 | 4.0 | 4.0 | 4.0 | 4.0 | 4.0 | 4.0 |
| (6) Lipophilic glycerin mono- stearate | | 2.0 | 2.0 | 2.0 | 2.0 | 2.0 | 2.0 | 2.0 | 2.0 |
| (7) POE Sorbitan mono-lauric acid ester (20E.O.) | | 2.0 | 2.0 | 2.0 | 2.0 | 2.0 | 2.0 | 2.0 | 2.0 |
| (8) Purified maslinic acid 1 | | 0.01 | | | | | | | |
| (9) Purified maslinic acid 2 | | | 0.01 | | | | | | |
| (9) Purified maslinic acid 2 | | | | 0.001 | | | | | |
| (10) Erythrodiol | | | | | 0.01 | | | | |
| (11) Uvaol | | | | | | 0.01 | | | |
| (12) Betulinic acid | | | | | | | 0.01 | | |
| (13) Betulin | | | | | | | | 0.01 | |
| (14) Comp. Of Synthesis 1 | | | | | | | | | 0.01 |
| (20) Preservative | | 0.2 | 0.2 | 0.2 | 0.2 | 0.2 | 0.2 | 0.2 | 0.2 |
| (21) Perfume | | q.s. (as much as suffices) | | | | | | | |
| (22) Purified water | | Balance | | | | | | | |
| Whitening effect | Effective | 13 | 14 | 10 | 12 | 13 | 13 | 12 | 14 |
| | Slightly effective | 2 | 1 | 4 | 3 | 1 | 1 | 3 | 1 |
| | Not effective | 0 | 0 | 1 | 0 | 1 | 1 | 0 | 0 |

Table 3 (continued)

| Component (%) | | Product of the invention | | | Comparative product | | | |
|---|---|---|---|---|---|---|---|---|
| | | 9 | 10 | 11 | 1 | 2 | 3 | 4 |
| (1) Bees wax | | 6.0 | 6.0 | 6.0 | 6.0 | 6.0 | 6.0 | 6.0 |
| (2) Cetanol | | 5.0 | 5.0 | 5.0 | 5.0 | 5.0 | 5.0 | 5.0 |
| (3) Reduced lanolin | | 5.0 | 5.0 | 5.0 | 5.0 | 5.0 | 5.0 | 5.0 |
| (4) Squalane | | 30.0 | 30.0 | 30.0 | 30.0 | 30.0 | 30.0 | 30.0 |
| (5) Glycerin monostearate | | 4.0 | 4.0 | 4.0 | 4.0 | 4.0 | 4.0 | 4.0 |
| (6) Lipophilic glycerin mono- stearate | | 2.0 | 2.0 | 2.0 | 2.0 | 2.0 | 2.0 | 2.0 |
| (7) POE Sorbitan mono-lauric acid ester (20 E.O.) | | 2.0 | 2.0 | 2.0 | 2.0 | 2.0 | 2.0 | 2.0 |
| (15) Comp. Of Synthesis 2 | | 0.01 | | | | | | |
| (16) Comp. Of Synthesis 3 | | | 0.01 | | | | | |
| (17) Comp. Of Synthesis 4 | | | | 0.01 | | | | |
| (18) Vitamin C-magnesium phosphate | | | | | 0.01 | | | |
| (19) Kojic acid | | | | | | 0.01 | | |
| (19) Kojic acid | | | | | | | 0.001 | |
| (20) Preservative | | 0.2 | 0.2 | 0.2 | 0.2 | 0.2 | 0.2 | 0.2 |
| (21) Perfume | | q.s. (as much as suffices) | | | | | | |
| (22) Purified water | | Balance | | | | | | |
| Whitening Effect | Effective | 13 | 13 | 13 | 3 | 12 | 5 | 0 |
| | Slightly effective | 2 | 2 | 2 | 8 | 3 | 6 | 1 |
| | Not effective | 0 | 0 | 0 | 4 | 0 | 4 | 14 |

(Preparation Method)

A. The components (1) to (6) and (20) were admixed together, heated to 70°C and then maintained at that temperature.
B. One of the components (7) to (19) and (22) was added to and mixed together, heated to 70°C and then maintained at that temperature.
C. The mixture A was added to and mixed with the mixture B.
D. The component (21) was added to the resulting mixture with cooling to give each corresponding cream.

(Test Method)

An appropriate amount of each cream to be tested was applied to the faces of female panelists (15 panelists of 27-year-old to 54-year-old per test cream) twice a day in the morning and evening every day over 12 weeks, after washing the faces. The whitening effect of each test cream thus applied to the face was evaluated according to the following evaluation criteria:

(Evaluation Criteria)

| Rank | Details |
|---|---|
| Effective | The test sample makes the darkening and dullness of the skin inconspicuous. |
| Slightly Effective | The test sample makes the darkening and dullness of the skin inconspicuous to some extent. |
| Not Effective | The conditions of the skin are almost identical to those observed before the test sample is applied. |

From the results listed in the foregoing Table 3, it has been proved that the cream of the present invention can reduce or prevent the occurrence of, for instance, any darkening and dullness on the skin and can make the skin beautiful. Moreover, it has also been proved that the whitening agent of the present invention possesses favorable whitening effect even at a low content as compared with the existing excellent whitening agent.

[Prescription 2]: Toilet Water

A toilet water having the following formulation was prepared according to the method specified below.

| Formulation | (%) |
|---|---|
| (1) Glycerin | 5.0 |
| (2) 1,3-Butylene glycol | 6.5 |
| (3) Polyoxyethylene (POE; 20 E.O.) sorbitan monolauric acid ester | 1.2 |
| (4) Ethanol | 8.0 |
| (5) Purified maslinic acid 1 | 0.2 |
| (6) Preservative | q.s. |
| (7) Perfume | q.s. |
| (8) Purified water | Balance |

(Preparation Method)

A. The components (3), (4), (6) and (7) were admixed and dissolved together.
B. The components (1), (2), (5) and (8) were admixed and dissolved together.
C. The resulting mixtures A and B were uniformly admixed together to form a toilet water.

[Prescription 3]: Milky Lotion

A milky lotion having the following formulation was prepared according to the following method.

| Formulation | (%) |
|---|---|
| (1) POE (10 E.O.) sorbitan monostearate | 1.0 |
| (2) POE (60 E.O.) sorbit tetraoleate | 0.5 |
| (3) Glycerin monostearate | 1.0 |
| (4) Stearic acid | 0.5 |
| (5) Behenyl alcohol | 0.5 |
| (6) Squalane | 8.0 |

(continued)

| Formulation | (%) |
|---|---|
| (7) Purified maslinic acid 2 | 0.01 |
| (8) Tocopherol acetic acid ester | 1.0 |
| (9) Preservative | 0.1 |
| (10) Carboxy vinyl polymer | 0.1 |
| (11) Sodium hydroxide | 0.05 |
| (12) Ethyl alcohol | 5.0 |
| (13) Purified water | Balance |
| (14) Perfume | q.s. |

(Preparation Method)

A. The components (9) to (13) were admixed together with heating and the resulting mixture was maintained at 70°C.
B. The components (1) to (6) were admixed together with heating and the resulting mixture was maintained at 70°C.
C. The mixture A was added to and mixed with the mixture B to thus give a uniform emulsion.
D. After cooling the product obtained in C, the components (7), (8) and (14) were added thereto and uniformly admixed together to give a milky lotion.

It was confirmed that both of the toilet water of Prescription 2 and the milky lotion of Prescription 3 were excellent in stability with time, that they could prevent any occurrence of darkening and dullness of the skin and relieve dye-precipitation such as melasma and that they could make the skin transparent and beautiful.

[Prescription 4]: Pack

A pack having the following formulation was prepared according to the following method.

| Formulation | (%) |
|---|---|
| (1) Polyvinyl alcohol | 20.0 |
| (2) Ethanol | 20.0 |
| (3) Glycerin | 5.0 |
| (4) Kaolin | 6.0 |
| (5) Purified maslinic acid 1 | 0.05 |
| (6) Preservative | q.s. |
| (7) Perfume | q.s. |
| (8) Purified water | Balance |

(Preparation Method)

A. The components (1), (3), (4) and (8) were admixed together, the resulting mixture was heated to 70°C and then stirred.
B. The components (2), (6) and (7) were admixed together.
C. The foregoing mixture B was added to and mixed with the foregoing mixture A, the resulting mixture was cooled and then the component (5) was uniformly dispersed in the mixture to give a pack.

It has been proved that the pack of Prescription 4 is excellent in stability with time and that the application thereof to the skin permits the conditioning of the texture of the skin, the prevention of any occurrence of darkening and dullness of the skin and the relief of the dye-precipitation such as melasma and makes the skin transparent and beautiful

[Prescription 5]: Liquid Foundation

A liquid foundation having the following formulation was prepared according to the following method.

| Formulation | (%) |
|---|---|
| (1) Lanolin | 7.0 |
| (2) Liquid paraffin | 5.0 |
| (3) Stearic acid | 2.0 |
| (4) Cetanol | 1.0 |
| (5) Glycerin | 5.0 |
| (6) Triethanolamine | 1.0 |
| (7) Carboxymethyl cellulose | 0.7 |
| (8) Purified water | Balance |
| (9) Mica | 15.0 |
| (10) Talc | 6.0 |
| (11) Titanium oxide | 3.0 |
| (12) Coloring pigment | 6.0 |
| (13) Purified maslinic acid 2 | 0.01 |
| (14) Ethyl p-methoxy-silicate | q.s. |
| (15) Perfume | q.s. |

(Preparation Method)

A. The components (1) to (4) were mixed and dissolved together.
B. The components (9) to (12) were added to and uniformly admixed with the foregoing mixture A.
C. The components (5) to (8) were uniformly dissolved together and the resulting mixture was maintained at 70°C.
D. The foregoing mixture C was added to and uniformly admixed with the foregoing mixture B to give an emulsion.
E. After cooling the foregoing mixture D, the components (13) to (15) were added thereto to give a liquid foundation.

[Prescription 6]: Sunscreen Milky Lotion

A sunscreen milky lotion having the following formulation was prepared according to the method detailed below.

| Formulation | (%) |
|---|---|
| (1) Stearic acid | 2.0 |
| (2) Cetanol | 1.0 |
| (3) Polyoxyethylene sorbitan (20 E.O.) mono-oleate | 0.5 |
| (4) Sorbitan sesqui-oleate | 0.5 |
| (5) 2-Ethylhexyl p-methoxy cinnamate | 8.0 |

(continued)

| Formulation | (%) |
|---|---|
| (6) Cetyl 2-ethylhexanoate | 12.0 |
| (7) 1,3-Butylene glycol | 10.0 |
| (8) Carboxy vinyl polymer | 0.2 |
| (9) Triethanolamine | 0.5 |
| (10) Purified maslinic acid 2 | 0.01 |
| (11) Purified water | Balance |
| (12) Preservative | q.s. |
| (13) Titanium oxide | 3.0 |
| (14) Perfume | q.s. |

(Preparation Method)

A. The components (1) to (6) were mixed together with heating and the resulting mixture was maintained at 75°C.
B. The components (7) to (12) were mixed together with heating and the resulting mixture was maintained at 75°C.
C. The foregoing mixture A was gradually added to the mixture B.
D. The components (13) to (14) were added to the resulting mixture C while cooling the latter to give a sunscreen milky lotion.

It has been proved that both of the liquid foundation of Prescription 5 and the sunscreen milky lotion of Prescription 6 were excellent in stability with time and that the application thereof to the skin could prevent the occurrence of any dark skin, melasma and ephelis ofthe skin.

[Prescription 7]: Gel Ointment

A gel ointment having the formulation specified below was prepared according to the method detailed below.

| Formulation | (%) |
|---|---|
| (1) Carboxy vinyl polymer | 1.0 |
| (2) Triethanolamine | 1.0 |
| (3) 1,3-Butylene glycol | 10.0 |
| (4) Purified maslinic acid 2 | 0.01 |
| (5) Purified water | Balance |

(Preparation Method)

A. The components (1) and (3) to (5) were admixed and dissolved together.
B. The component (2) was added to the resulting mixture A and uniformly mixed together to give a gel ointment.

It has been proved that the gel ointment of Prescription 7 is excellent in stability with time and that the application thereof to the skin permits the conditioning of the texture of the skin, the prevention of any occurrence of darkening and dullness of the skin and the relief of the dye-precipitation such as melasma and can make the skin transparent and beautiful.

[Prescription 8]: Milky Lotion

A milky lotion having the formulation specified below was prepared according to the method detailed below.

| Formulation | (%) |
|---|---|
| (1) Polyoxyethylene (10 E.O.) sorbitan monostearate | 1.0 |
| (2) Polyoxyethylene (60 E.O.) sorbit tetraoleate | 0.5 |
| (3) Glyceryl monostearate | 1.0 |
| (4) Stearic acid | 0.5 |
| (5) Behenyl alcohol | 0.5 |
| (6) Squalane | 8.0 |
| (7) Compound of Synthesis 2 | 0.01 |
| (8) Tocopherol acetate | 1.0 |
| (9) Preservative | 0.1 |
| (10) Carboxy vinyl polymer | 0.1 |
| (11) Sodium hydroxide | 0.05 |
| (12) Ethyl alcohol | 5.0 |
| (13) Purified water | Balance |
| (14) Perfume | q.s. |

(Preparation Method)

A. The components (9) to (13) were admixed together with heating and the resulting mixture was maintained at 70°C.
B. The components (1) to (6) were admixed together with heating and the resulting mixture was maintained at 70°C.
C. The foregoing mixture B was added to and uniformly admixed with the foregoing mixture A to give an emulsion.
D. After cooling the resulting mixture C, the components (7), (8) and (14) were added thereto and uniformly mixed together to give a milky lotion.

It has been proved that the milky lotion of Prescription 8 is excellent in stability with time and that the application thereof to the skin permits the prevention of any occurrence of darkening and dullness of the skin and the relief of the dye-precipitation such as melasma and can make the skin transparent and beautiful.

[Prescription 9]: Sunscreen Milky Lotion

A sunscreen milky lotion having the following formulation was prepared according to the following method.

| Formulation | (%) |
|---|---|
| (1) Stearic acid | 2.0 |
| (2) Cetanol | 1.0 |
| (3) Polyoxyethylene sorbitan monooleate (20 E.O.) | 0.5 |
| (4) Sorbitan sesqui-oleate | 0.5 |
| (5) 2-Ethylhexyl p-methoxy-silicate | 8.0 |
| (6) Cetyl 2-ethylhexanoate | 12.0 |
| (7) 1,3-Butylene glycol | 10.0 |

(continued)

| Formulation | (%) |
|---|---|
| (8) Carboxy vinyl polymer | 0.2 |
| (9) Triethanolamine | 0.5 |
| (10) Compound of Synthesis 7 | 0.01 |
| (11) Purified water | Balance |
| (12) Preservative | q.s. |
| (13) Titanium oxide | 3.0 |
| (14) Perfume | q.s. |

(Preparation Method)

A. The components (1) to (6) were admixed together with heating and the resulting mixture was maintained at 75°C.
B. The components (7) to (12) were admixed together with heating and the resulting mixture was maintained at 75°C.
C. The foregoing mixture A was gradually added to the foregoing mixture B.
D. The components (13) to (14) were added to the resulting mixture C, while cooling the latter to thus give a sunscreen milky lotion.

It has been proved that the sunscreen milky lotion of Prescription 9 is excellent in stability with time and that the application thereof to the skin can prevent the occurrence of any dark skin, melasma and ephelis of the skin due to, for instance, sunburn.

[Prescription 10]: Toilet Water

A toilet water having the following formulation was prepared according to the method specified below.

| Formulation | (%) |
|---|---|
| (1) Glycerin | 5.0 |
| (2) 1,3-Butylene glycol | 6.5 |
| (3) Polyoxyethylene (20 E.O.) sorbitan monolauric acid ester | 1.2 |
| (4) Ethanol | 8.0 |
| (5) 3-O- β -D-Glucopyranosyl-maslinic acid | 0.2 |
| (6) Preservative | q.s. |
| (7) Perfume | q.s. |
| (8) Purified water | Balance |

(Preparation Method)

A. The components (3), (4), (6) and (7) were admixed and dissolved together.
B. The components (1), (2), (5) and (8) were admixed and dissolved together.
C. The resulting mixtures A and B were uniformly admixed together to form a toilet water.

It has been proved that the toilet water of Prescription 10 is excellent in stability with time and that the application thereof to the skin permits the prevention of any occurrence of darkening and dullness of the skin and the relief

of the dye-precipitation such as melasma and can make the skin transparent and beautiful.

The present invention permits the preparation of an external agent for the skin and a whitening agent having a strong whitening effect and low cytotoxicity. Moreover, penta-cyclic triterpenes or maslinic acid, erythrodiol, uvaol, betulinic acid, betulin and physiologically acceptable salts or derivatives thereof have excellent whitening effects and low cytotoxicity and therefore, the use thereof permits the preparation of an external agent for the skin and a whitening agent having an excellent whitening effect. In particular, maslinic acid and/or physiologically acceptable salts thereof can be obtained from plants such as olive plants and therefore, the present invention would permit the stable supply of whitening components, which are safe for the skin and have quite strong whitening effects. In addition, the whitening components can be obtained from by products formed during the oil expression steps and therefore, the price thereof may be reduced. Maslinic acid, erythrodiol, uvaol, betulinic acid, betulin and physiologically acceptable salts or derivatives thereof, as penta-cyclic triterpenes, according to the present invention show a strong whitening effect even at a trace amount and therefore, they may be used in various fields such as cosmetics, pharmaceutical agents and quasi-drugs. Moreover, they do not adversely affect the skin and accordingly, they can favorably be used in cosmetics.

## Claims

**1.** An external agent for the skin comprising a compound selected from the group consisting of maslinic acid, erythrodiol, uvaol, betulinic acid, betulin, physiologically acceptable salts thereof and derivatives thereof.

**2.** An external agent for the skin comprising the following components (A) and (B):

(A) A compound selected from the group consisting of maslinic acid, erythrodiol, uvaol, betulinic acid, betulin, physiologically acceptable salts thereof and derivatives thereof;
(B) At least one pharmaceutical agent selected from the group consisting of whitening agents, antioxidants, anti-inflammatory agents, cell-activators, ultraviolet screening agents, blood circulation promoters and humectants.

**3.** The external agent for the skin of claim 1 wherein the compounds selected from the group consisting of maslinic acid, erythrodiol, uvaol, betulinic acid, betulin, physiologically acceptable salts thereof and derivatives thereof are those isolated from naturally occurring materials.

**4.** An external agent for the skin comprising maslinic acid and/or physiologically acceptable salts thereof.

**5.** An external agent for the skin comprising the following components (A) and (B):

(A) A compound selected from the group consisting of maslinic acid and/or physiologically acceptable salts thereof;
(B) At least one pharmaceutical agent selected from the group consisting of whitening agents, antioxidants, anti-inflammatory agents, cell-activators, ultraviolet screening agents, blood circulation promoters and humectants.

**6.** The external agent for the skin as set forth in any one of claims 1 to 5, wherein the maslinic acid and/or physiologically acceptable salts thereof are derived from naturally occurring materials selected from the group consisting of olive, hop, mint, pomegranate, clove, sage or jujube.

**7.** The external agent for the skin as set forth in any one of claims 1 to 6, wherein the maslinic acid and/or physiologically acceptable salts thereof are those prepared by extracting olive plants and/or products obtained during the olive oil production processes with water and/or an organic solvent and then subjecting the resulting extract to concentration and/or fractionation-purification treatments.

**8.** The external agent for the skin of claim 7 wherein the organic solvent is a hydrophilic organic solvent.

**9.** The external agent for the skin of claim 7 wherein the organic solvent is an alcohol.

**10.** The external agent for the skin of claim 7 wherein the organic solvent is ethanol.

**11.** The external agent for the skin of claim 7 wherein the total content of maslinic acid and physiologically acceptable

salts thereof present in the mixture of maslinic acid and/or physiologically acceptable salts thereof prepared by the method as set forth in claim 7 is not less than 95% by mass.

**12.** The external agent for the skin of claim 1 wherein it comprises the compound selected from the group consisting of maslinic acid, erythrodiol, uvaol, betulinic acid, betulin, physiologically acceptable salts thereof and derivatives thereof in an amount ranging from 0.00001 to 10% by mass on the basis of the total mass of the external agent for the skin.

**13.** A whitening agent comprising, as an effective component, a compound selected from the group consisting of maslinic acid, erythrodiol, uvaol, betulinic acid, betulin, physiologically acceptable salts thereof and derivatives thereof.

**14.** The whitening agent of claim 13 wherein the compounds selected from the group consisting of maslinic acid, erythrodiol, uvaol, betulinic acid, betulin, physiologically acceptable salts thereof and derivatives thereof are those isolated from naturally occurring materials.

**15.** A whitening agent comprising, as an effective component, maslinic acid and/or physiologically acceptable salts thereof.

**16.** The whitening agent as set forth in claim 13 or 15, wherein the maslinic acid and/or physiologically acceptable salts thereof are derived from naturally occurring materials selected from the group consisting of olive, hop, mint, pomegranate, clove, sage or jujube.

**17.** The whitening agent as set forth in claim 15 wherein the maslinic acid and/or physiologically acceptable salts thereof are those prepared by extracting olive plants and/or products obtained during the olive oil production processes with water and/or an organic solvent and then subjecting the resulting extract to concentration and/or fractionation-purification treatments.

**18.** The whitening agent as set forth in claim 17 wherein the organic solvent is a hydrophilic organic solvent.

**19.** The whitening agent as set forth in claim 17 wherein the organic solvent is an alcohol.

**20.** The whitening agent as set forth in claim 17 wherein the organic solvent is ethanol.

**21.** The whitening agent as set forth in claim 17 wherein the total content of maslinic acid and physiologically acceptable salts thereof present in the mixture of maslinic acid and/or physiologically acceptable salts thereof prepared by the method as set forth in claim 17 is not less than 95% by mass.

**22.** The whitening agent as set forth in claim 13 wherein it comprises the compound selected from the group consisting of maslinic acid, erythrodiol, uvaol, betulinic acid, betulin, physiologically acceptable salts thereof and derivatives thereof in an amount of not less than 0.001% by mass on the basis of the total mass of the whitening agent.

**23.** A method of using a compound selected from the group consisting of maslinic acid, erythrodiol, uvaol, betulinic acid, betulin, physiologically acceptable salts thereof and derivatives thereof, as an external agent for the skin.

**24.** A method of using a compound selected from the group consisting of maslinic acid, erythrodiol, uvaol, betulinic acid, betulin, physiologically acceptable salts thereof and derivatives thereof, as a whitening agent.

**25.** A melanogenesis-inhibitory agent comprising, as an effective component, a compound selected from the group consisting of maslinic acid, erythrodiol, uvaol, betulinic acid, betulin, physiologically acceptable salts thereof and derivatives thereof.

**26.** A method for preventing any occurrence of the dark skin, melasma, ephelis or darkening and dullness of the skin, comprising the step of using of a compound selected from the group consisting of maslinic acid, erythrodiol, uvaol, betulinic acid, betulin, physiologically acceptable salts thereof and derivatives thereof

**27.** Use of a compound selected from the group consisting of maslinic acid, erythrodiol, uvaol, betulinic acid, betulin, physiologically acceptable salts thereof and derivatives thereof, as an external agent for the skin.

**28.** Use of a compound selected from the group consisting of maslinic acid, erythrodiol, uvaol, betulinic acid, betulin, physiologically acceptable salts thereof and derivatives thereof, as a whitening agent.

**29.** Use of a compound selected from the group consisting of maslinic acid, erythrodiol, uvaol, betulinic acid, betulin, physiologically acceptable salts thereof and derivatives thereof, as a melanogenesis-inhibitory agent.

**30.** A raw material for a whitening agent comprising a compound selected from the group consisting of maslinic acid, erythrodiol, uvaol, betulinic acid, betulin, physiologically acceptable salts thereof and derivatives thereof.

**31.** Use of a compound selected from the group consisting of maslinic acid, erythrodiol, uvaol, betulinic acid, betulin, physiologically acceptable salts thereof and derivatives thereof, as a raw material for a whitening agent.

**32.** A method for preparing an external agent for the skin, which comprises maslinic acid, physiologically acceptable salts thereof and/or derivatives thereof, wherein the method comprises the steps of derivatizing, if necessary, maslinic acid, physiologically acceptable salts thereof and/or derivatives thereof, which are prepared by extracting olive plants and/or products obtained during the olive oil production processes with water and/or an organic solvent and then subjecting the resulting extract to concentration and/or fractionation-purification treatments, and

incorporating, into the resulting product, at least one oil component or medicinal oil and at least one nonionic surfactant.

**33.** A method for preparing a toilet water, which comprises maslinic acid, physiologically acceptable salts thereof and/or derivatives thereof, wherein the method comprises the step of blending maslinic acid, physiologically acceptable salts thereof and/or derivatives thereof, which are prepared by extracting olive plants and/or products obtained during the olive oil production processes with water and/or an organic solvent and then subjecting the resulting extract to concentration and/or fractionation-purification treatments, with at least one alcohol.

## INTERNATIONAL SEARCH REPORT

International application No.
PCT/JP01/02787

A. CLASSIFICATION OF SUBJECT MATTER
Int.Cl[7] A61K 7/00, 7/48, A61P 17/16

According to International Patent Classification (IPC) or to both national classification and IPC

B. FIELDS SEARCHED

Minimum documentation searched (classification system followed by classification symbols)
Int.Cl[7] A61K 7/00-7/50

Documentation searched other than minimum documentation to the extent that such documents are included in the fields searched

Electronic data base consulted during the international search (name of data base and, where practicable, search terms used)
CA (STN), REGISTRY (STN), MEDLINE (STN), WPI (DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y | JP, 11-292752, A (Lion Corporation), 26 October, 1999 (26.10.99), entire description; especially, column 8, lines 1 to 9; columns 14 to 24; working example (Family: none) | 1,2,12,13,22, 25,30,31<br>3-11,14-21, 32,33 |
| X | JP, 10-265328, A (Nippon Flour Mills Co., Ltd.), 06 October, 1998 (06.10.98), entire description; especially, working examples 5, 6 (Family: none) | 1,3 |
| X | JP, 9-67253, A (Lion Corporation), 11 March, 1997 (11.03.97), entire description; especially, column 3, lines 2 to 5; composition example 3 (Family: none) | 1,3 |
| X | JP, 10-152444, A (Nippon Flour Mills Co., Ltd.), 09 June, 1998 (09.06.98), entire description; especially, column 3 (Family: none) | 1,3 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 May, 2001 (30.05.01) | 12 June, 2001 (12.06.01) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP01/02787

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | JP, 10-25236, A (Pola Chemical Industries Inc.), 27 January, 1998 (27.01.98), entire description; especially working examples (Family: none) | 1-3,12-14,22, 25,30,31<br>4-11,15-21, 32,33 |
| X | EP, 717983, A1 (Unilever PLC), 26 June, 1996 (26.06.96), entire description<br>& EP, 717983, B1 & US, 5529769, A<br>& CA, 2164050, A & JP, 8-208424, A<br>& ZA, 9510138, A & DE, 69520029, E | 1,3 |
| X | WO, 98/00093, A1 (Pola Chemical Industries Inc.), 08 January, 1998 (08.01.98), entire description<br>& JP, 9-143050, A & AU, 9719421, A<br>& KR, 2000022300, A & US, 6207711, B1 | 1,3 |
| X | JP, 9-87156, A (Pola Chemical Industries Inc.), 31 March, 1997 (31.03.97), entire description; especially, working examples (Family: none) | 1,3 |
| Y | JP, 58-57307, A (Pola Chemical Industries Inc.), 05 April, 1983 (05.04.83), entire description<br>& JP, 2-2848, B2 | 1-22,25, 30-33 |
| Y | JP, 7-149622, A (Shiseido Company, Limited), 13 June, 1995 (13.06.95), entire description (Family: none) | 1-22,25, 30-33 |
| Y | BIANCHI, G. et al., "Pentacyclic Triterpene Acids in Olives," Phytochemistry, Vol. 37, No. 1, (1994), pages 205 to 207 | 1-22,25, 30-33 |
| Y | RECIO, M. C. et al., "Structual Requirements for the Anti-Inflammatory Activity of Natural Triterpenoids," Planta Med., Vol. 61, (1995), pages 182 to 185 | 1-22,25, 30-33 |
| Y | Manez, S. et al., "Effect of Selected Triterpenoids on chronic dermal inflammation", European Journal of Pharmacology, Vol.334, (1997), pages 103 to 105 | 1-22,25, 30-33 |

Form PCT/ISA/210 (continuation of second sheet) (July 1992)

**INTERNATIONAL SEARCH REPORT**

International application No.
PCT/JP01/02787

**Box I Observations where certain claims were found unsearchable (Continuation of item 1 of first sheet)**

This international search report has not been established in respect of certain claims under Article 17(2)(a) for the following reasons:

1. ☒ Claims Nos.: 23,24,26-29
because they relate to subject matter not required to be searched by this Authority, namely:

The inventions of claims 23, 24, and 26-29 include methods for treatment of the human body by therapy (see, e.g., the disclosure " can be formulated into pharmaceutical preparations for external use including ..." in lines 18-19 and 24 of page 51 of the description).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)